# EUROPEAN PATENT APPLICATION

(11) **EP 4 529 923 A1**
(43) Date of publication of application: **02.04.2025**
(21) Application number: 23811641.2
(22) Date of filing: 11.05.2023
(51) Int. Cl.: A61K 9/20, A61K 47/02, A61K 47/04, A61K 47/12, A61K 47/30, A61K 31/167, A61P 25/02, A61P 29/00

(54) **GEL COATING AND ORAL SOLID COMPOSITION**

(30) Priority: 23.05.2022 JP 2022084151; 26.04.2023 JP 2023072747; 26.04.2023 JP 2023072748; 26.04.2023 JP 2023072749
(71) Applicant: Kyowa Pharmaceutical Industry Co., Ltd., Osaka-shi Osaka 530-0005 (JP)
(72) Inventor: Shingaki Hiroki, Sanda-shi Hyogo 669-1324 (JP); Asai Shota, Sanda-shi Hyogo 669-1324 (JP); Taniguchi Fumiyasu, Sanda-shi Hyogo 669-1324 (JP); Yamashita Junya, Sanda-shi Hyogo 669-1324 (JP)
(74) Representative: Kraus & Lederer PartGmbB
(86) International application number: PCT/JP2023/017747
(87) International publication number: WO 2023/228766

(57) **Abstract**

By coating an oral solid preparation with a gel film containing a polymer gelable on contact with water, a carbonate, and an acidic substance, obtained oral solid composition is easy to swallow, and elution of active ingredients is suppressed, while the composition can disintegrate in the oral cavity. When an oral solid composition comprising a gel film is hermetically packed or is tightly packed together with a desiccant, discoloration during storage can be suppressed. In a gel film, by setting the concentration of glycerol at 2% by mass or less relative to the total amount of the gel film, mold is prevented from growing. In the case where a coating liquid in which a polymer gelable on contact with water is dispersed is used to form a gel film, the gel film can be formed for a short period of time, even when the coating liquid contains a large amount of the polymer. A gel fil containing titanium oxide at 20% by mass or less can be identifiably marked by ultraviolet laser irradiation.

## Description

### Technical Field

The present invention relates to a gel film, an oral solid composition comprising the gel film, and a coating liquid and a coating composition for forming the gel film.

### Background Art

Oral solid preparations such as tablets and capsules put burden on elderly people with difficulty in swallowing, children with difficulty in taking tablets, dry mouse patients, or other people, and this may reduce the adherence to result in insufficient treatment effect.

To help patients with difficulty in swallowing solid preparations to take the preparations, orally disintegrating tablets that can disintegrate in the oral cavity with a small amount of water or without water and is easily swallowed have been widely used.

The orally disintegrating tablets, however, are likely to leave ingredients in the pharyngeal region, and a predetermined amount of the drug may fail to be absorbed. A method of suppressing bitterness in the oral cavity is adopted to bitter drug-containing orally disintegrating tablets since orally disintegrating tablets are disintegrated in the oral cavity. For example, the drug itself or granules containing the drug are coated to mask the bitterness, or an artificial sweetener is added to correct the taste. The coating involves complicated processing, and thus the production of orally disintegrating tablets is costly. When a sweetening agent is used, an artificial sweetener such as sucralose or aspartame is often used, and bitterness characteristic of the artificial sweetener may remain in the oral cavity.

Meanwhile, to support medication for patients with difficulty in swallowing solid preparations, medication jellies are commercially available. Using a medication jelly together with a solid preparation such as tablets helps swallowing the solid preparation. It is, however, recommended to take a pharmaceutical product only with water. When a solid preparation is taken together with a commercially available medication jelly, the medication jelly may interfere with elution of an active ingredient in the gastrointestinal tract, and insufficient treatment effect may be achieved.

Coating a solid preparation with a jelly film has been proposed.

For example, Patent Literature 1 teaches that by coating the surface of capsules, tablets, or the like with a water-soluble polymer that is lubricated with water, swallowing is helped.

Patent Literature 2 teaches that by coating the surface of micropellets with an outer layer containing a swelling agent, a homogeneous dispersion is formed when the micropellets come in contact with water, and no individual grains can be perceived.

Patent Literature 3 teaches that by providing a coating layer containing a water-soluble polymer on the surface of capsules, tablets, or other preparations and by further providing, on the outside thereof, a salivation promoting agent, the coating layer becomes a viscous mass when the preparation comes into contact with saliva or water, and the preparation does not adhere to the mucosa in the oral cavity.

Patent Literature 4 teaches that by coating particles containing a sugar alcohol with a layer containing a gelling agent that exhibits slidability when coming into contact with water, taking the particles is helped.

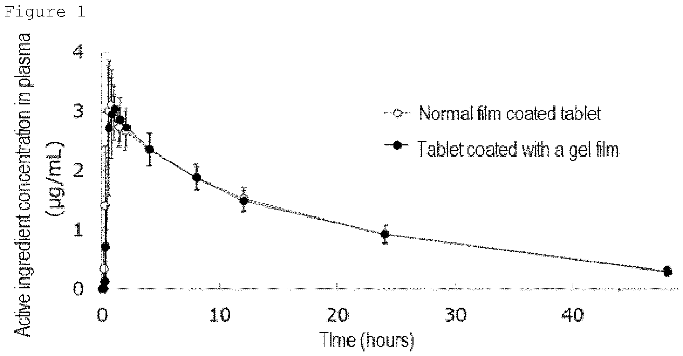

In the solid preparations in Patent Literatures 1 to 4, however, a jelly film that helps taking a preparation interferes with elution of a drug in the gastrointestinal tract. For example, Patent Literature 4 shows that by coating particles containing an active ingredient with a layer containing a gelling agent, elution of the active ingredient was delayed in water (Fig. 1). Therefore, such a solid preparation may achieve insufficient treatment effect as with the combination use with a swallowing aid jelly.

Patent Literature 5 discloses a solid preparation that solves the problems. In the solid preparation, a solid pharmaceutical preparation is coated with an undercoat containing a water-soluble polymer and with a gel coat containing a polymer gelable on contact with water, in this order. The undercoat contains a carbonate, and the gel coat contains an acidic substance. Alternatively, the undercoat contains an acidic substance, and the gel coat contains a carbonate. In the mouth, the gel coat gelates by contact with water to help swallowing. After swallowing, the water-soluble polymer in each coat is dissolved in water, and the carbonate and the acidic substance react each other to cause foaming. Accordingly, the coating layers disappear in the gastrointestinal tract, and the solid pharmaceutical preparation rapidly elutes a drug.

Of pharmaceutical products and food supplements, solid preparations such as tablets and capsules are individually packaged in a PTP (press through package) or a SP (strip package), typically. In a PTP, tablets or the like are placed in plastic concave portions, and the concave portions are sealed with an aluminum sheet. By pressing the concave portion, the tablet or the like can be taken out. In an SP, tablets or the like are placed in a film-like bag, and the bag is sealed by heat sealing. These packages are simple and hygienic and provides moisture barrier properties to some extent, and thus the packaging techniques are often used.

A pharmaceutical product or a food supplement with a large single dose is often packaged in a bottle for sale. A bottled product eliminates the labor of taking out each tablet and thus is suitable for preparations that are taken for a long period of time or are taken frequently a day.

When a solid preparation having a surface with a gel film is stored, mold is likely to grow on the surface. Such a preparation thus has disadvantages in requiring careful packaging or careful control of temperature, humidity, and other conditions during storage.

To further improve the ease of swallowing of a solid preparation having a surface with a gel film, a larger amount of a polymer gelable on contact with water may be added. However, when a larger amount of the polymer gelable on contact with water is added, the coating liquid has a higher viscosity and may clog nozzles of a coating apparatus. When a larger amount of a solvent is added to match the amount of the thickener, the coating liquid has an appropriate viscosity and is prevented from clogging nozzles of the coating apparatus, but the amount of the coating liquid becomes larger, and coating takes a longer time.

On solid preparations such as tablets and capsules, an identification code or a trade name is typically displayed for correct handling during production or dispensing and for correct dosing. In particular, when displayed, the trade name can be identified even after the solid preparation is taken out of a PTP package, and this can reduce dispensing errors or dosing errors. However, many small characters are displayed for a trade name and may not be clearly displayed by engraving in many cases.

The technique for clearly displaying small characters include inkjet printing, thermal transfer printing, and laser irradiation. Of them, laser irradiation has advantages such as easily printing even on a curved surface and noncontact printing. This technique uses color tone changes caused when ultraviolet laser light is applied to titanium oxide (TiO₂). A solid preparation is coated with a coating layer containing titanium oxide, and ultraviolet laser light is applied. Accordingly, characters are displayed on the coating layer.

### Citation List

### Patent Literature

Patent Literature 1: JP1986-161215A
Patent Literature 2: JP1988-196511A
Patent Literature 3: JP2000-516222A
Patent Literature 4: WO2017/057147
Patent Literature 5: JP6964369B

### Summary of Invention

### Technical Problem

The present invention has a first objective to provide a gel film on an oral solid preparation (in the present invention, a film containing a polymer gelable on contact with water is called a "gel film"). The gel film helps swallowing the oral solid preparation and suppresses the delay in elution of an active ingredient from the oral solid preparation. The first objective of the present invention is also to provide an oral solid composition comprising a gel film on an oral solid preparation. The oral solid composition is easily swallowed, and the delay in elution of an active ingredient from the oral solid preparation is suppressed.

The inventors of the present invention have found that in an oral solid composition comprising an oral solid preparation coated with a gel film, the gel film is likely to discolor during storage. The present invention thus has a second objective to provide an oral solid composition comprising a gel film, and the gel film is prevented from discoloring during storage.

The present invention has a third objective to provide a gel film that can be formed for a short period of time even when a larger amount of the polymer is contained, and mold is difficult to grow on the gel film. The third objective of the present invention is also to provide an oral solid composition comprising a gel film. The gel film can be formed for a short period of time even when a larger amount of a polymer gelable on contact with water is used, and mold is difficult to grow on the surface of the gel film.

The inventors of the present invention have found that ultraviolet laser irradiation can display characters identifiably on a typical coating layer but is difficult to display characters identifiably on a gel film.

The present invention thus has a fourth objective to provide a gel film having a surface on which characters or the like are clearly displayed without engraving or any coloring agent. The fourth objective of the present invention is also to provide an oral solid composition comprising a gel film. On the surface of the gel film, characters or the like are clearly displayed without engraving or any coloring agent.

### Solution to Problem

The inventors of the present invention have repeated studies to achieve the above objectives and have revealed the following findings.
(1) By coating an oral solid preparation with a gel film containing a polymer gelable on contact with water, a carbonate, and an acidic substance, the gel coat gelates by contact with water in the mouth to help swallowing. After swallowing, the water-soluble polymer is dissolved in water, and the carbonate and the acidic substance react with each other to cause foaming. Accordingly, the gel film disappears in the gastrointestinal tract, and the oral solid preparation rapidly elutes an active ingredient.
(2) When an oral solid composition comprising a gel film is packaged in a packing material that is typically used for pharmaceutical products or food supplements, the gel film is likely to discolor during storage, but when the oral solid composition is hermetically packed or is tightly packed together with a desiccant, discoloration during storage can be effectively suppressed.
(3-1) In a gel film, by setting the concentration of glycerol at 2% by mass or less relative to the total amount of the gel film, mold is prevented from growing.
(3-2) In the case where a coating liquid in which a polymer gelable on contact with water is dispersed is used to coat an oral solid preparation, the coating liquid does not have a high viscosity and does not clog nozzles of a coating apparatus, even when the coating liquid contains a large amount of the polymer gelable on contact with water. In addition, a larger amount of a solvent to reduce the viscosity is not needed, and thus a gel film can be formed for a short period of time.
(4) When the surface of a gel film containing titanium oxide (TiO₂) at an excessively high concentration is irradiated with ultraviolet laser light to display characters or the like, excessively high concentration of the titanium oxide hinders display of the characters or the like. By setting the concentration of titanium oxide at 20% by mass or less relative to the total amount of the gel film, characters or the like can be displayed identifiably by ultraviolet laser irradiation.

The present invention has been completed on the basis of the above findings and provides the following [1-1] to [4-12].

### First embodiment

[1-1] A rapidly disintegrating gel film comprising a polymer gelable on contact with water, a carbonate, and an acidic substance.
[1-2] The rapidly disintegrating gel film according to [1-1], in which the carbonate is contained at 0.5 to 60% by mass relative to the total amount of the rapidly disintegrating gel film.
[1-3] The rapidly disintegrating gel film according to [1-1] or [1-2], in which the acidic substance is contained at 0.5 to 40% by mass relative to the total amount of the rapidly disintegrating gel film.
[1-4] The rapidly disintegrating gel film according to any one of [1-1] to [1-3], in which the acidic substance is contained at 0.1 to 1 part by mass relative to 1 part by mass of the carbonate.
[1-5] An oral solid composition comprising the rapidly disintegrating gel film according to any one of [1-1] to [1-4] and an oral solid preparation.
[1-6] The oral solid composition according to [1-5], in which the oral solid preparation is a tablet.
[1-7] A coating solid composition comprising a polymer gelable on contact with water, a carbonate, and an acidic substance.
[1-8] The coating solid composition according to [1-7], in which the polymer gelable on contact with water is a polysaccharide.
[1-9] The coating solid composition according to [1-7] or [1-8], in which the concentration of the polymer gelable on contact with water is 16 to 56% by mass relative to the total amount of the coating solid composition.
[1-10] The coating solid composition according to any one of [1-7] to [1-9], in which the concentration of the carbonate is 0.5 to 60% by mass relative to the total amount of the coating solid composition.
[1-11] The coating solid composition according to any one of [1-7] to [1-10], in which the concentration of the acidic substance is 0.5 to 40% by mass relative to the total amount of the coating solid composition.
[1-12] The coating solid composition according to any one of [1-7] to [1-11], in which the content of the acidic substance is 0.1 to 1 part by mass relative to 1 part by mass of the carbonate.
[1-13] Use of a solid composition comprising a polymer gelable on contact with water, a carbonate, and an acidic substance, as a coating composition.
[1-14] Use of a solid composition comprising a polymer gelable on contact with water, a carbonate, and an acidic substance, for producing a coating composition.

### Second embodiment

[2-1] An oral solid composition comprising a gel film containing a polymer gelable on contact with water and comprising an oral solid preparation, the oral solid composition being hermetically packed or being tightly packed together with a desiccant.
[2-2] The oral solid composition according to [2-1], in which the oral solid composition is hermetically packed in a plastic bottle, a bag molded from a plastic sheet, or a bag molded from a sheet comprising an aluminum layer or is tightly packed in a plastic bottle.
[2-3] The oral solid composition according to [2-1] or [2-2], in which the oral solid composition packaged in a PTP package or an SP package is hermetically packed or is tightly packed together with a desiccant.
[2-4] The oral solid composition according to [2-2] or [2-3], in which the aluminum layer of the bag molded from a sheet comprising an aluminum layer has a thickness of 5 to 40 µm.
[2-5] The oral solid composition according to any one of [2-1] to [2-4], in which the hermetically packed oral solid composition is hermetically packed together with a desiccant.
[2-6] The oral solid composition according to any one of [2-1] to [2-5], in which the polymer gelable on contact with water is a polysaccharide.
[2-7] The oral solid composition according to any one of [2-1] to [2-6], in which the oral solid preparation is a tablet.

### Third embodiment

[3-1] A gel film formed by using a coating liquid containing a polymer gelable on contact with water, in which the concentration of glycerol is 2% by mass or less relative to the total amount of the gel film, and the polymer gelable on contact with water is partially or practically completely dispersed in the coating liquid.
[3-2] The gel film according to [3-1], in which the polymer gelable on contact with water is a polysaccharide.
[3-3] The gel film according to [3-1] or [3-2], in which the coating liquid contains a polar solvent other than water.
[3-4] The gel film according to any one of [3-1] to [3-3], further comprising a carbonate and/or an acidic substance.
[3-5] An oral solid composition comprising the gel film according to any one of [3-1] to [3-4] and an oral solid preparation.
[3-6] The oral solid composition according to [3-5], in which the oral solid preparation is a tablet.

[3-7] A coating liquid comprising a polymer gelable on contact with water, in which the concentration of glycerol is 2% by mass or less relative to the total amount of the solid content in the coating liquid, and the polymer gelable on contact with water is partially or practically completely dispersed in the coating liquid.
[3-8] The coating liquid according to [3-7], in which the polymer gelable on contact with water is a polysaccharide.
[3-9] The coating liquid according to [3-7] or [3-8], in which the coating liquid contains a polar solvent other than water.
[3-10] The coating liquid according to any one of [3-7] to [3-9], further comprising a carbonate and/or an acidic substance.
[3-11] Use of a liquid comprising a polymer gelable on contact with water, in which the concentration of glycerol is 2% by mass or less relative to the total amount of the solid content in the coating liquid, and the polymer gelable on contact with water is partially or practically completely dispersed in the liquid, as a coating liquid.
[3-12] Use of a liquid comprising a polymer gelable on contact with water, in which the concentration of glycerol is 2% by mass or less relative to the total amount of the solid content in the coating liquid, and the polymer gelable on contact with water is partially or practically completely dispersed in the liquid, for producing a coating liquid.

### Fourth embodiment

[4-1] A gel film comprising a polymer gelable on contact with water and comprising titanium oxide (TiO₂) at 20% by mass or less relative to the total amount of the gel film, in which marking is provided on the surface of the gel film without engraving or any coloring agent.
[4-2] The gel film according to [4-1], in which the concentration of titanium oxide (TiO₂) in the gel film is 0.1% by mass or more relative to the total amount of the gel film.
[4-3] The gel film according to [4-1] or [4-2], in which the polymer gelable on contact with water is a polysaccharide.
[4-4] The gel film according to any one of [4-1] to [4-3], further comprising a carbonate and/or an acidic substance.
[4-5] The gel film according to any one of [4-1] to [4-4], in which the marking is provided on the surface of the gel film by ultraviolet laser irradiation.
[4-6] The gel film according to any one of [4-1] to [4-5], formed by using a coating liquid in which the polymer gelable on contact with water is partially or practically completely dispersed.
[4-7] An oral solid composition comprising the gel film according to any one of [4-1] to [4-6] and an oral solid preparation.
[4-8] The oral solid composition according to [4-7], in which the oral solid preparation is a tablet.
[4-9] The oral solid composition according to [4-7] or [4-8], in which marking is provided on the surface of the oral solid composition by ultraviolet laser irradiation.

[4-10] A coating composition for forming a gel film having a surface with marking, the coating composition comprising a polymer gelable on contact with water and comprising titanium oxide at 20% by mass or less relative to the total amount of the coating composition.
[4-11] The coating composition according to [4-10], in which the polymer gelable on contact with water is a polysaccharide.
[4-12] The coating composition according to [4-10] or [4-11], further comprising a carbonate and/or an acidic substance.
[4-13] Use of a composition comprising a polymer gelable on contact with water and comprising titanium oxide at 20% by mass or less relative to the total amount of the composition, as a coating composition for forming a gel film having a surface with marking.
[4-14] Use of a composition comprising a polymer gelable on contact with water and comprising titanium oxide at 20% by mass or less relative to the total amount of the composition, for producing a coating composition for forming a gel film having a surface with marking.

### Advantageous Effect of Invention

### First embodiment

When an oral solid composition in which an oral solid preparation is coated with the rapidly disintegrating gel film in the first embodiment of the present invention is taken, the water-soluble polymer contained in the gel film gelates with saliva or water and becomes smooth, and thus the oral solid composition is easily swallowed. In the gastrointestinal tract, the oral solid composition further absorbs water, and the water-soluble polymer is dissolved. Therefore, the gel film disintegrates. Accordingly, the carbonate and the acidic substance come into contact with each other to generate carbon dioxide gas as foaming, and the gel film disappears. Hence, the delay in elution of an active ingredient from the oral solid preparation due to the presence of the gel film is not caused or is hardly caused. In the oral solid composition, a carbonate and an acidic substance are contained in the same layer, but the composition is easily swallowed and thus is unlikely to stay in the mouth. Hence, in the mouth, the gel film hardly disintegrates, and an active ingredient is practically not eluted.

In the oral solid composition, a carbonate and an acidic substance are contained in one layer, but the storage stability is good, and the gel film is prevented from deteriorating or disintegrating during storage.

The gel film can be formed by a single coating operation, and thus the oral solid composition is easily produced at low costs and is highly practical.

### Second embodiment

An oral solid composition comprising a gel film is likely to discolor during storage when packaged in a PTP package or an SP package, which is typically used as a package for tablets, capsules, or the like, but the oral solid composition in the second embodiment of the present invention is hermetically packed or is tightly packed together with a desiccant, and thus the gel film is prevented from discoloring.

### Third embodiment

In the gel film in the third embodiment of the present invention, the concentration of glycerol is suppressed at 2% by mass or less, and thus mold is difficult to grow. Hence, the gel film can be distributed or stored under common conditions, and accordingly, special controls can be eliminated. Even when a package typically used for oral solid compositions is used, mold is difficult to grow.

The gel film in the third embodiment is formed by coating an oral solid preparation with a coating liquid in which a polymer gelable on contact with water is dispersed. Even when containing the polymer in a large amount, the coating liquid does not have an excessively high viscosity. Accordingly, the coating liquid is prevented from clogging nozzles of a coating apparatus during coating. Increasing the amount of a solvent to reduce the concentration of the polymer in the coating liquid is not needed, and thus a gel film can be formed for a short period of time.

### Fourth embodiment

On the gel film in the fourth embodiment of the present invention, characters or the like are identifiably displayed without engraving or any coloring agent by a simple method of adding titanium oxide at an appropriate concentration. Displaying characters or the like, that is, marking, can be performed by ultraviolet laser irradiation, and thus small characters or the like can be identifiably displayed, for example, with high contrast.

The gel film can be marked by ultraviolet laser irradiation, and thus characters or the like can be displayed on a curved surface of a tablet or a capsule. Thermal transfer printing may decompose ingredients, and inkjet printing may smudge tablets with inks by contact between the tablets. Ultraviolet laser irradiation can discolor an ingredient of the gel film to form marking, and thus the gel film has no such concern as above.

When an oral solid composition in which an oral solid preparation is coated with the gel film in the fourth embodiment is taken, the water-soluble polymer contained in the gel film gelates with saliva or water and becomes smooth, and thus the oral solid composition is easily swallowed.

### Brief Description of Drawings

Figure 1 is a figure showing change in time of active ingredient concentration in the plasma of a patient who is orally administered a tablet having a gel film or a tablet having a usual film coating layer.
Figure 2 is photographs showing mold growth states of the tablets of Example 3-1 in the third embodiment, and Comparative Examples 3-1 and 3-2**.**
Figure 3 is photographs showing marking states of the surfaces of tablets of Examples 4-1 to 4-6 in the fourth embodiment and Comparative Examples 4-1 to 4-4**.**

### Description of embodiments

### (1) First embodiment

### (1-1) Rapidly disintegrating gel film

The rapidly disintegrating gel film in the first embodiment of the present invention is a gel film containing a polymer gelable on contact with water, a carbonate, and an acidic substance. The gel film is a single layer containing the polymer gelable on contact with water, the carbonate, and the acidic substance.

### Polymer gelable on contact with water

Examples of the polymer gelable on contact with water include polysaccharides such as xanthan gum, karaya gum, locust bean gum, tragacanth gum, guar gum, tamarind seed gum, tara gum, gum arabic, sodium alginate, pregelatinized starch, carrageenan, gellan gum, dextran, dextrin, pullulan, galactomannan, agar, and derivatives thereof; vinyl polymers such as carboxyvinyl polymer; proteins such as casein and gelatin.

Of them, the polysaccharide and the vinyl polymer are preferred, and sodium alginate, carboxyvinyl polymer, and xanthan gum are more preferred, and xanthan gum is even more preferred.

The Polymer gelable on contact with water may be used singly or in combination of two or more of them.

The concentration of the polymer gelable on contact with water can be 16% by mass or more, 22% by mass or more, 28% by mass or more, or 34% by mass or more relative to the total amount of the gel film. Within the above range, the gel film is easily swallowed. The concentration of the polymer gelable on contact with water can be 56% by mass or less, 50% by mass or less, 44% by mass or less, or 38% by mass or less relative to the total amount of the gel film. Within the above range, the gel film becomes less sticky.

The concentration of the polymers gelable on contact with water relative to the total amount of the gel film may be 16 to 56% by mass, 16 to 50% by mass, 16 to 44% by mass, 16 to 38% by mass, 22 to 56% by mass, 22 to 50% by mass, 22 to 44% by mass, 22 to 38% by mass, 28 to 56% by mass, 28 to 50% by mass, 28 to 44% by mass, 28 to 38% by mass, 34 to 56% by mass, 34 to 50% by mass, 34 to 44% by mass, or 34 to 38% by mass, for example.

### Carbonate

Any of a water-soluble carbonate and a water-insoluble or poorly water-soluble carbonate can be used as a carbonate. Examples of the water-soluble carbonate include sodium carbonate, potassium carbonate, sodium hydrogen carbonate, potassium hydrogen carbonate, and ammonium carbonate. Examples of the water-insoluble or poorly water-soluble carbonate include calcium carbonate and magnesium carbonate. Of them, the water-soluble carbonate is preferred, a hydrogen carbonate is more preferred, and sodium hydrogen carbonate and potassium hydrogen carbonate are even more preferred.

Carbonates may be used singly or in combination of two or more of them.

The carbonate of the present invention includes a carbonate which is not used for the purpose of generating carbonic acid.

The concentration of the carbonate can be 0.5% by mass or more relative to the total amount of the rapidly disintegrating gel film, and is preferably 1% by mass or more, more preferably 3% by mass or more, even more preferably 5% by mass or more, further more preferably 9% by mass or more. Within the above range, carbonic acid can be generated such a degree that the gel film disintegrates in the digestive tract. The concentration of the carbonate is preferably 60% by mass or less, more preferably 50% by mass or less, even more preferably 40% by mass or less, further more preferably 30% by mass or less relative to the total amount of the rapidly disintegrating gel film. The concentration of the carbonate can also be 25% by mass or less, or 20% by mass or less. Within the above range, generation of carbonic acid and subsequent disintegration of the gel film before dosing an oral solid composition having the gel film are suppressed.

The concentration of the carbonate may be 0.5 to 60 % by mass, 0.5 to 50 % by mass, 0.5 to 40 % by mass, 0.5 to 30 % by mass, 0.5 to 25 % by mass, 0.5 to 20 % by mass, 1 to 60 % by mass, 1 to 50 % by mass, 1 to 40 % by mass, 1 to 30 % by mass, 1 to 25 % by mass, 1 to 20 % by mass, 3 to 60 % by mass, 3 to 50 % by mass, 3 to 40 % by mass, 3 to 30 % by mass, 3 to 25 % by mass, 3 to 20 % by mass, 5 to 60 % by mass, 5 to 50 % by mass, 5 to 40 % by mass, 5 to 30 % by mass, 5 to 25 % by mass, 5 to 20 % by mass, 9 to 60 % by mass, 9 to 50 % by mass, 9 to 40 % by mass, 9 to 30 % by mass, 9 to 25 % by mass, or 9 to 20 % by mass, for example.

### Acid Substance

The acid substance is a substance that reacts with the carbonate and generates carbon dioxide.

Examples of the acid substance include organic acids such as adipic acid, ascorbic acid, L-aspartic acid, L-glutamic acid, benzoic acid, erythorbic acid, acetic acid, tartaric acid, fumaric acid, phthalic acid, malonic acid, citric acid, malic acid, lactic acid, maleic acid, tannic acid, and salicylic acid; inorganic acids such as hydrochloric acid, and phosphoric acid. Of them, the organic acid is preferred. The acid substance can be a hydrate.

The acid substances may be used singly or in combination of two or more of them.

The acid substance of the present invention includes an acid substance which is not used for the purpose of generating carbonic acid.

Concentration of the acid substance is preferably 0.5% by mass or more, more preferably 1% by mass or more, even more preferably 3% by mass or more, further more preferably 5% by mass or more relative to the total amount of the rapidly disintegrating gel film. Within the above range, carbonic acid can be generated such a degree that the gel film disintegrates in the digestive tract.

The concentration of the acid substance is preferably 40% by mass or less, more preferably 30% by mass or less, even more preferably 20% by mass or less relative to the total amount of the rapidly disintegrating gel film. It can be 15% by mass or less. Within the above range, generation of carbonic acid and subsequent disintegration of the gel film before dosing an oral solid composition having the gel film are suppressed.

The concentration of the acid substance may be 0.5 to 40 % by mass, 0.5 to 30 % by mass, 0.5 to 20 % by mass, 0.5 to 15 % by mass, 1 to 40% by mass, 1 to 30 % by mass, 1 to 20% by mass, 1 to 15 % by mass, 3 to 40 % by mass, 3 to 30% by mass, 3 to 20 % by mass, 3 to 15 % by mass, 5 to 40 % by mass, 5 to 30 % by mass, 5 to 20 % by mass, or 5 to 15 % by mass, for example.

The content of the acid substance is preferably 0.1 part by mass or more, more preferably 0.3 part by mass or more, even more preferably 0.45 part by mass or more relative to 1 part by mass of the carbonate. The content is preferably 1 part by mass or less, more preferably 0.7 part by mass or less, and even more preferably 0.5 part by mass or less. Within the above range, carbonic acid can be generated such a degree that the gel film disintegrates in the digestive tract.

The content of the acid substance relative to 1 part by mass of the carbonate may be 0.1 to 1 part by mass,0.1 to 0.7 part by mass, 0.1 to 0.5 part by mass, 0.3 to 1 part by mass, 0.3 to 0.7 part by mass, 0.3 to 0.5 part by mass, 0.45 to 1 part by mass, 0.45 to 0.7 part by mass, or 0.45 to 0.5 part by mass, for example.

Examples of preferred combinations of the carbonate and the acid substance include the combination of sodium carbonate with ascorbic acid, the combination of sodium carbonate with citric acid, the combination of sodium carbonate with tartaric acid, the combination of sodium carbonate with malic acid, the combination of sodium carbonate with aspartic acid, the combination of sodium carbonate with glutamic acid, the combination of potassium carbonate with ascorbic acid, the combination of potassium carbonate with citric acid, the combination of potassium carbonate with tartaric acid, the combination of potassium carbonate with malic acid, the combination of potassium carbonate with aspartic acid, the combination of potassium carbonate with glutamic acid, the combination of sodium hydrogen carbonate with ascorbic acid, the combination of sodium hydrogen carbonate with citric acid, the combination of sodium hydrogen carbonate with tartaric acid, the combination of sodium hydrogen carbonate with malic acid, the combination of sodium hydrogen carbonate with aspartic acid, the combination of sodium hydrogen carbonate with glutamic acid, the combination of potassium hydrogen carbonate with ascorbic acid, the combination of potassium hydrogen carbonate with citric acid, the combination of potassium hydrogen carbonate with tartaric acid, the combination of potassium hydrogen carbonate with malic acid, the combination of potassium hydrogen carbonate with aspartic acid, the combination of potassium hydrogen carbonate with glutamic acid, the combination of ammonium carbonate with ascorbic acid, the combination of ammonium carbonate with citric acid, the combination of ammonium carbonate with tartaric acid, the combination of ammonium carbonate with malic acid, the combination of ammonium carbonate with aspartic acid, the combination of ammonium carbonate with glutamic acid.

In these combinations, ascorbic acid, citric acid, tartaric acid, malic acid, aspartic acid, and glutamic acid can be a hydrate.

### Excipients

Any excipients generally usable in the coating layer of an oral solid formulation can be added to the gel film of the present invention as long as it does not impair the effect of the present invention.

Examples of the excipients include a diluent, a binder, a disintegrant, a lubricant, a fluidizer, a brightening agent, a stabilizer, an antioxidant, an emulsifier, a surfactant, a solubilizer, a suspending agent, buffer, a pH adjuster, a thickener, an adsorption agent, a coloring agent, a corrigent, a sweetening agent, a flavoring agent, a preservative or antiseptic, a foaming agent, a defoaming agent, a solvent, a water-soluble coating agent, and a plasticizer. The excipients may be used singly or in combination of two or more of them.

Following examples of the excipient include a substance that corresponds to a polymer gelating by contact with water, which means that such a substance functions as the following agent beside a gelator.

Examples of the diluent include erythritol, lactose/microcrystalline cellulose spheres, powdered candy, gum arabic, powdered gum arabic, pregelatinized starch, partially pregelatinized starch, starch (wheat starch, rice starch, potato starch, corn starch), isomalt hydrate (ISOMALT), kaolin, reduced palatinose, xylitol, L-glutamine, croscarmellose sodium, crospovidone, silica-treated microcrystalline cellulose, magnesium silicate, light anhydrous silicic acid, microcrystalline cellulose, synthetic aluminum silicate, synthetic hydrotalcite, titanium oxide, β- cyclodextrin, aluminum hydroxide gel, refined white sugar, refined white sugar spherical granule, gelatin, D-sorbitol, talc, medium-chain triglyceride, precipitated calcium carbonate, low substituted hydroxypropyl cellulose, sodium starch glycolate (sodium carboxymethyl starch), trehalose hydrate, lactose hydrate, white soft sugar, white soft sugar/starch spherical granule, hydroxypropyl starch, hydroxypropyl cellulose, hypromellose, glucose, pullulan, polyoxyethylene hydrogenated castor oil (N), polyoxyethylene (N) polyoxypropylene (N) glycol, macrogols (macrogol 4000, macrogol 6000), maltitol, D-mannitol, lactose anhydrous, calcium hydrogen phosphate anhydrous, magnesium aluminate metasilicate, glycerol monostearate, calcium monohydrogen phosphate, and calcium hydrogen phosphate hydrate.

Examples of the binder include gum arabic, powdered gum arabic, pregelatinized starch, partially pregelatinized starch, starch (wheat starch, rice starch, corn starch, potato starch), sodium alginate, sodium carboxymethyl cellulose, calcium carboxymethyl cellulose, gelatin, low-substituted hydroxypropyl cellulose, dextrin, sodium starch glycolate (sodium carboxymethyl starch), hydroxypropyl starch, hydroxypropyl cellulose, hypromellose, pullulan, macrogols (macrogol 400, macrogol 4000, macrogol 6000), ethyl acrylate/methyl methacrylate copolymer dispersion, aminoalkyl methacrylate copolymer (such as aminoalkyl methacrylate copolymer E), ammonioalkyl methacrylate copolymer (aminoalkyl methacrylate copolymer RS), ethyl cellulose, carboxy vinyl polymer, carboxymethyl ethyl cellulose, sodium carboxymethyl ethyl cellulose, calcium carboxymethyl ethyl cellulose, powdered agar, guar gum, copolyvidone, cetanol, shellac, dextrin, hydroxyethylcellulose, hypromellose acetate succinate, hypromellose phthalate, pectin, povidone, polyvinyl alcohol, polyvinyl acetal diethylaminoacetate, polyvinyl alcohol/acrylic acid/methyl methacrylate copolymer, polyvinyl alcohol(partially saponified product), polyvinyl alcohol/polyethylene glycol graft copolymer, methacrylic acid copolymer (dried methacrylic acid copolymer LD, methacrylic acid copolymer L, methacrylic acid copolymer LD, methacrylic acid copolymer S), methylcellulose, and ethyl cellulose.

Examples of the disintegrant include starch (wheat starch, rice starch, potato starch, corn starch), sodium starch glycolate (sodium carboxymethyl starch), hydroxypropyl starch, hydroxypropylcellulose, partially pregelatinized starch, croscarmellose sodium, crospovidone, light anhydrous silicic acid, microcrystalline cellulose, synthetic aluminum silicate, magnesium aluminometasilicate, carmellose, carmellose calcium, calcium silicate, and sodium lauryl sulfate.

Examples of the lubricant include talc, glycerin monostearate, dimethylpolysiloxane (for internal use), sucrose fatty acid ester, stearic acid, stearates (calcium stearate, magnesium stearate, sodium stearyl fumarate), and dl-leucine.

Examples of the fluidizer include calcium silicate, talc, light anhydrous silicic acid, synthetic aluminum silicate, and magnesium aluminometasilicate.

Examples of the brightening agent include carnauba wax, purified paraffin/carnauba wax-mixed wax, white beeswax, and purified shellac.

Examples of the stabilizer include meglumine, citric acid hydrate, sodium benzoate, sodium edetate hydrate, dibutylhydroxytoluene, xylitol, D-sorbitol, lactose hydrate, D-mannitol, sodium citrate hydrate, tartaric acid, anhydrous citric acid, DL-malic acid, talc, light anhydrous silicic acid, magnesium aluminometasilicate, glycerol monostearate, sucrose fatty acid ester, stearic acid, sodium lauryl sulfate, macrogols (such as macrogol 400 and macrogol 4000), carboxyvinyl polymer, polyvinyl alcohol (partially saponified products), polyoxyethylene hydrogenated castor oil (N), L-aspartic acid, sodium L-aspartate hydrate, DL-alanine, L-alanine, L-arginine, sodium chloride, dried aluminum hydroxide gel, xanthan gum, glycine, acetic acid, sodium acetate hydrate, sodium hydroxide, sodium hydrogen carbonate, tocopherol, lactic acid, concentrated glycerin, butylated hydroxyanisole, fumaric acid, propylene glycol, propyl gallate, polysorbate 80, and anhydrous sodium monohydrogen phosphate.

Examples of the antioxidant include anhydrous citric acid, citric acid hydrate, soybean lecithin, dibutylhydroxytoluene, tocopherol, and propyl gallate.

Examples of the emulsifier include glycerol monostearate, sodium lauryl sulfate, polyoxyethylene hydrogenated castor oil (N), polysorbate 80, medium-chain triglyceride, soybean lecithin, and lauromacrogol.

Examples of the surfactant include glycerol monostearate, sodium lauryl sulfate, polyoxyethylene hydrogenated castor oil (N), polysorbate 80, lauromacrogol, macrogols (such as macrogol 400), and polyoxyethylene (N) polyoxypropylene (N) glycol.

Examples of the solubilizer include sodium lauryl sulfate, polyoxyethylene hydrogenated castor oil (N), polysorbate 80, lauromacrogol, polyoxyethylene (N) polyoxypropylene (N) glycol, medium-chain triglyceride, soybean lecithin, meglumine, D-mannitol, sodium citrate hydrate, anhydrous citric acid, sucrose fatty acid ester, macrogols (such as macrogol 4000 and macrogol 6000), polyvinyl alcohol (partially saponified products), L-aspartic acid, L-arginine, sodium hydroxide, sodium hydrogen carbonate, lactic acid, concentrated glycerin, hydroxypropyl cellulose, β-cyclodextrin, glycerol, soybean oil, and triacetin.

Examples of the suspending agent include polyoxyethylene hydrogenated castor oil (N), polysorbate 80, soybean lecithin, sucrose fatty acid ester, macrogols (such as macrogol 4000 and macrogol 6000), sodium hydroxide, hydroxypropyl cellulose, glycerol, D-sorbitol, magnesium aluminometasilicate, carboxyvinyl polymer, dried aluminum hydroxide gel, xanthan gum, butylated hydroxyanisole, propylene glycol, microcrystalline cellulose, gum arabic, powdered gum arabic, hypromellose, powdered agar, povidone, methyl cellulose, kaolin, carrageenan, carmellose sodium, glycerol fatty acid ester, and magnesium aluminum silicate.

Examples of the buffer include sodium citrate hydrate, anhydrous citric acid, sodium hydrogen carbonate, lactic acid, citric acid hydrate, sodium benzoate, tartaric acid, DL-malic acid, sodium chloride, acetic acid, sodium acetate hydrate, anhydrous sodium monohydrogen phosphate, L-glutamic acid, and diluted hydrochloric acid.

Examples of the pH adjuster include L-glutamine, sodium citrate hydrate, anhydrous citric acid, sodium hydrogen carbonate, lactic acid, citric acid hydrate, tartaric acid, DL-malic acid, acetic acid, sodium acetate hydrate, anhydrous sodium monohydrogen phosphate, diluted hydrochloric acid, sodium hydroxide, meglumine, succinic acid, and aqueous ammonia.

Examples of the thickener include guar gum, hydroxypropyl cellulose, carboxyvinyl polymer, xanthan gum, propylene glycol, hypromellose, carrageenan, carmellose sodium, concentrated glycerin, gelatin, hydroxyethyl cellulose, carob bean gum, α-cyclodextrin, and locust bean gum.

Examples of the adsorption agent include magnesium aluminometasilicate, kaolin, light anhydrous silicic acid, synthetic aluminum silicate, magnesium silicate, and precipitated calcium carbonate.

Examples of the coloring agent include titanium oxide, indigocarmine, yellow ferric oxide, carmine, black iron oxide, red ferric oxide, synthetic food dyes (such as Food Blue No.1, Food Blue No.2 aluminum lake, Food Yellow No.4, Food Yellow No.4 aluminum lake, Food Yellow No.5, Food Red No.2, Food Red No.3, and Food Red No.102), and natural food dyes.

Examples of the corrigent include erythritol, xylitol, sucrose, D-sorbitol, lactose hydrate, white soft sugar, glucose, D-mannitol, aspartame, powdered cacao, hydrogenated maltose starch syrup, hydrogenated sugar syrup, glycyrrhiza, glycyrrhiza extract, citric acid hydrate, sodium citrate hydrate, L-glutamic acid, succinic acid, saccharin, saccharin sodium hydrate, tartaric acid, sucralose, purified stevia extract, peppermint oil, anhydrous citric acid, l-menthol, and DL-malic acid.

Examples of the sweetening agent include xylitol, purified sucrose, D-sorbitol, lactose hydrate, sucrose, glucose, D-mannitol, aspartame, hydrogenated maltose starch syrup, glycyrrhiza, glycyrrhiza extract, saccharin, saccharin sodium hydrate, sucralose, purified stevia extract, maltitol, acesulfame potassium, and thaumatin.

Examples of the flavoring agent include peppermint oil, l-menthol, and vanillin.

Examples of the preservative or antiseptic include citric acid hydrate, sodium benzoate, sodium edetate hydrate, dibutylhydroxytoluene, and p-hydroxybenzoate esters (such as isobutyl p-hydroxybenzoate, isopropyl p-hydroxybenzoate, ethyl p-hydroxybenzoate, butyl p-hydroxybenzoate, propyl p-hydroxybenzoate, and methyl p-hydroxybenzoate).

Examples of the foaming agent include hydrogen carbonates (such as sodium hydrogen carbonate and potassium hydrogen carbonate) and carbonates (such as magnesium carbonate and calcium carbonate).

Examples of the defoaming agent include dimethyl polysiloxane (for internal use).

Examples of the solvent include ethanol and water.

The polymer gelable on contact with water is a water-soluble polymer. The gel film in the first embodiment of the present invention may contain, in addition to the water-soluble polymer gelable on contact with water, a water-soluble polymer not gelable even on contact with water and is commonly used as a water-soluble coating agent for an oral solid preparation.

Examples of such a water-soluble polymer include cellulose coating agents such as methyl cellulose, hydroxyethyl cellulose, hydroxypropyl cellulose, hydroxypropyl methyl cellulose (hypromellose), hydroxypropyl cellulose phthalate, carboxymethyl ethyl cellulose, carboxymethyl cellulose, sodium carboxymethyl cellulose, hypromellose phthalate, hypromellose acetate succinate, cellulose acetate, and cellulose acetate phthalate; vinyl polymers such as carboxyvinyl polymers, polyvinyl alcohols, povidone, copolyvidone, and polyvinyl alcohol-polyethylene glycol copolymers; polyoxyethylene-polyoxypropylene glycol; polyethylene glycol (macrogol); and methacrylate polymers or methacrylic acid polymers.

Examples of the methacrylate polymer or the methacrylic acid polymer include aminoalkyl methacrylate copolymer E (such as Eudragits (registered trademark) E100 and EPO), methacrylic acid copolymer LD (such as Eudragits L30D-55 and L100-55), methacrylic acid copolymer L (such as Eudragit L100), and methacrylic acid copolymer S (such as Eudragit S100), each manufactured by Roehm.

Of them, cellulose coating agents and vinyl polymers are preferred; hydroxypropyl cellulose, hydroxypropyl methyl cellulose, and povidone are more preferred; and hydroxypropyl methyl cellulose is even more preferred.

When water-soluble polymer not gelable even on contact with water along with the polymergelable on contact with water is contained, the concentration of the polymer gelable on contact with water relative to the total amount of these polymers may be 19% by mass or more, 28% by mass or more, or 37% by mass or more, and may be 61% by mass or less, 53% by mass or less, or 43% by mass or less.

The concentration of the polymer gelable on contact with water relative to the total amount of the water-soluble polymer not gelable even on contact with water and the polymer gelable on contact with water may be 19 to 61 % by mass, 19 to 52 % by mass, 19 to 43 % by mass, 28 to 61 % by mass, 28 to 52 % by mass, 28 to 43 % by mass, 37 to 61 % by mass, 37 to 52 % by mass, 37 to 43 % by mass, for example.

Examples of the plasticizer include polyethylene glycol, propylene glycol, glycerin, glycerol fatty acid esters such as triacetin (glycerol triacetate), liquid paraffin, sorbitan monolaurate, glycerol monostearate, triethyl citrate, tributyl citrate, diethyl phthalate, dibutyl phthalate, diethyl sebacate, dibutyl sebacate, poloxamer, polyoxyethylene hardened castor oil.

The gel film may not contain pullulan. If the gel film contains pullulan, the concentration of pullulan may be less than 0.5% by mass.

The gel film may not contain any sugar alcohols, especially erythritol. If the gel film contains sugar alcohol, the concentration of sugar alcohol may be less than 40% by mass or more than 90% by mass.

The gel film may not contain any polyvalent metal compounds.

### Thickness of film

The thickness of the rapidly disintegrating gel film can be 0.02mm or more, 0.03mm or more, 0.04 mm or more, or 0.05 mm or more, and can be 0.4 mm or less, 0.3 mm or less, 0.2 mm or less, or 0.1 mm or less. Within the above range, the gel film is easily swallowed, and an active ingredient in an oral solid formulation can be rapidly released. When the film consists of multiple rapidly disintegrating gel films, the thickness of a film is total thickness of them.

The thickness of the rapidly disintegrating gel film may be 0.02 to 0.4 mm, 0.02 to 0.3 mm, 0.02 to 0.2 mm, 0.02 to 0.1 mm, 0.03 to 0.4 mm, 0.03 to 0.3 mm, 0.03 to 0.2 mm, 0.03 to 0.1 mm, 0.04 to 0.4 mm, 0.04 to 0.3 mm, 0.04 to 0.2 mm, 0.04 to 0.1 mm, 0.05 to 0.4 mm, 0.05 to 0.3 mm, 0.05 to 0.2 mm, or 0.05 to 0.1 mm, for example.

### (1-2) Oral solid composition

The oral solid composition in the first embodiment of the present invention is a composition comprising the above-mentioned rapidly disintegrating gel film in the first embodiment and an oral solid preparation. The oral solid composition in the first embodiment can be a composition in which an oral solid preparation is coated with the above-mentioned rapidly disintegrating gel film in the first embodiment.

The oral solid preparation is not specifically limited, and examples include powders, granules, fine granules, dry syrups, capsules (hard capsules and soft capsules), tablets, films, electuaries, and chewing gums. Examples also include, as special tablets, orally disintegrating tablets, chewable tablets, foam tablets, dispersible tablets, soluble tablets, and troches. Particularly preferred are tablets that are difficult to swallow.

The rapidly disintegrating gel film in the first embodiment does not interfere or hardly interferes with elution of ingredients from an oral solid composition, and thus the oral solid composition may contain any active ingredient. A single active ingredient may be contained, or two or more active ingredients may be contained. Hence, the oral solid composition can be widely used as a pharmaceutical composition, a quasi-drug composition, or a food composition.

The oral solid preparation may have a maximum diameter of 0.1 to 20 mm.

The ratio of mass of the gel film which shows the thickness of the gel film coating the oral solid formulation relative to the total amount of the oral solid formulation can be 0.1% by mass or more, 0.5% by mass or more, 1% by mass or more, or 2% by mass or more. The ratio can be 30% by mass or less, 20% by mass or less, 10% by mass or less, or 7% by mass or less. Within the above range, the gel film is easily swallowed, and an active ingredient in the oral solid formulation can be rapidly released.

The ratio of mass of the gel film relative to the total amount of the oral solid formulation may be 0.1 to 30% by mass, 0.1 to 20% by mass, 0.1 to 10% by mass, 0.1 to 7% by mass, 0.5 to 30% by mass, 0.5 to 20% by mass, 0.5 to 10% by mass, 0.5 to 7% by mass, 1 to 30% by mass, 1 to 20% by mass, 1 to 10% by mass, 1 to 7% by mass, 2 to 30% by mass, 2 to 20% by mass, 2 to 10% by mass, or 2 to 7% by mass, for example.

The oral solid composition in the first embodiment may comprise a plurality of rapidly disintegrating gel films having the same formulation or different formulations. When a plurality of rapidly disintegrating gel films is included, the total thickness of them can be within the above range.

Between the rapidly disintegrating gel film and the oral solid preparation and/or on the outside of the rapidly disintegrating gel film, one or more water-soluble polymer-containing layers may be provided. The water-soluble polymer-containing layer other than the rapidly disintegrating gel film may contain a carbonate or an acidic substance or may contain neither a carbonate nor an acidic substance. When an additional water-soluble polymer-containing layer is provided between the rapidly disintegrating gel film and the oral solid preparation, the stability of an active ingredient unstable to acids or bases is improved. The gel film gives sticky feeling, but when an additional water-soluble polymer-containing layer is provided on the outside of the rapidly disintegrating gel film, the resulting oral solid composition has no sticky feeling.

As the water-soluble polymer in the water-soluble polymer layer, for example, the polymer not gelable even on contact with water and has been exemplified as the water-soluble coating agent (water-soluble polymer) that can be contained in the rapidly disintegrating gel film can be used. The polymer gelable on contact with water is also a water-soluble polymer and thus can be used. As the excipient containable in the water-soluble polymer layer, for example, excipients exemplified as the excipients containable in the rapidly disintegrating gel film can be used. Water-soluble polymers and excipients may be contained singly or in combination of two or more of them.

To coat an oral solid preparation with the gel film, coating can be performed in a common procedure by using a machine such as a fluidized-bed coating machine, a Wurster fluidized-bed coating machine, a centrifugal fluidized-bed coating machine, a tumbling fluidized-bed coating machine, and a pan coating machine.

### (1-3) Coating solid composition

The first embodiment of the present invention encompasses a coating solid composition containing a polymer gelable on contact with water, a carbonate, and an acidic substance. The composition can be used as a coating solid composition for coating an oral solid preparation. The coating solid composition in the first embodiment contains substantially no solvent and specifically contains no solvent. The coating solid composition is thus a solid composition in the form of powder, granules, blocks, or the like. By coating an oral solid preparation with a coating liquid prepared by mixing the coating solid composition with a solvent, an oral solid composition comprising a gel film can be produced. The present invention therefore encompasses a method for producing an oral solid composition comprising a gel film. The method comprises a step of coating an oral solid preparation with a coating liquid prepared by mixing the coating solid composition in the first embodiment with a solvent.

The types of the polymer gelable on contact with water, the carbonate, the acidic substance, and the containable excipients are as described for the gel film.

The concentration of the polymers gelable on contact with water can be 16% by mass or more, 22% by mass or more, 28% by mass or more, or 34% by mass or more relative to the total amount of the coating solid composition. Within the above range, the gel film easily swallowed can be formed. The concentration of the polymer gelable on contact with water can be 56% by mass or less, 50% by mass or less, 44% by mass or less, or 38% by mass or less relative to the total amount of the coating solid composition. Within the above range, the gel film becomes less sticky.

The concentration of the polymers gelable on contact with water relative to the total amount of the coating solid composition may be 16 to 56 % by mass, 16 to 50 % by mass, 16 to 44 % by mass, 16 to 38 % by mass, 22 to 56 % by mass, 22 to 50 % by mass, 22 to 44 % by mass, 22 to 38 % by mass, 28 to 56 % by mass, 28 to 50 % by mass, 28 to 44 % by mass, 28 to 38 % by mass, 34 to 56 % by mass, 34 to 50 % by mass, 34 to 44 % by mass, or 34 to 38 % by mass, for example.

When water-soluble polymer not gelable on contact with water along with the polymer gelable on contact with water is contained, the concentration of the polymer gelable on contact with water relative to the total amount of these water-soluble polymers may be 19% by mass or more, 28% by mass or more, or 37% by mass or more. The concentration may be 61% by mass or less, 52% by mass or less, or 43% by mass or less.

The concentration of the polymer gelable on contact with water relative to the total amount of the water-soluble polymer not gelable even on contact with water and the polymer gelable on contact with water may be 19 to 61 % by mass, 19 to 52 % by mass, 19 to 43 % by mass, 28 to 61 % by mass, 28 to 52 % by mass, 28 to 43 % by mass, 37 to 61 % by mass, 37 to 52 % by mass, or 37 to 43 % by mass, for example.

The concentration of the carbonate can be 0.5% by mass or more relative to the total amount of the coating solid composition. The concentration is preferably 1% by mass or more, more preferably 3% by mass or more, even more preferably 5% by mass or more, further more preferably 9% by mass or more. Within the above range, carbonic acid can be generated such a degree that the gel film disintegrates in the digestive tract.

The concentration of the carbonate is preferably 60% by mass or less, more preferably 50% by mass or less, even more preferably 40% by mass or less, further more preferably 30% by mass or less relative to the total amount of the coating solid composition. The concentration can also be 25% by mass or less, or 20% by mass or less. Within the above range, generation of carbonic acid and subsequent disintegration of the gel film before dosing an oral solid composition having the gel film are suppressed.

The concentration of the carbonate relative to the total amount of the coating solid composition may be 0.5 to 60 % by mass, 0.5 to 50 % by mass, 0.5 to 40 % by mass, 0.5 to 30 % by mass, 0.5 to 25 % by mass, 0.5 to 20 % by mass, 1 to 60 % by mass, 1 to 50 % by mass, 1 to 40 % by mass, 1 to 30 % by mass, 1 to 25 % by mass, 1 to 20 % by mass, 3 to 60 % by mass, 3 to 50 % by mass, 3 to 40 % by mass, 3 to 30 % by mass, 3 to 25 % by mass, 3 to 20 % by mass, 5 to 60 % by mass, 5 to 50 % by mass, 5 to 40 % by mass, 5 to 30 % by mass, 5 to 25 % by mass, 5 to 20 % by mass, 9 to 60 % by mass, 9 to 50 % by mass, 9 to 40 % by mass, 9 to 30 % by mass, 9 to 25 % by mass, or 9 to 20 % by mass, for example.

Concentration of the acid substance is preferably 0.5% by mass or more, more preferably 1% by mass or more, even more preferably 3% by mass or more, further more preferably 5% by mass or more relative to the total amount of the coating solid composition. Within the above range, carbonic acid can be generated such a degree that the gel film disintegrates in the digestive tract.

The concentration of the acid substance is preferably 40% by mass or less, more preferably 30% by mass or less, even more preferably 20% by mass or less relative to the total amount of the coating solid composition. The concentration can be 15% by mass or less. Within the above range, generation of carbonic acid and subsequent disintegration of the gel film before dosing an oral solid composition having the gel film are suppressed.

The concentration of the acid substance relative to the total amount of the coating solid composition may be 0.5 to 40 % by mass, 0.5 to 30 % by mass, 0.5 to 20 % by mass, 0.5 to 15 % by mass, 1 to 40% by mass, 1 to 30 % by mass, 1 to 20% by mass, 1 to 15 % by mass, 3 to 40 % by mass, 3 to 30% by mass, 3 to 20 % by mass, 3 to 15 % by mass, 5 to 40 % by mass, 5 to 30 % by mass, 5 to 20 % by mass, or 5 to 15 % by mass, for example.

The content of the acid substance is preferably 0.1 part by mass or more, more preferably 0.3 part by mass or more, even more preferably 0.45 part by mass or more relative to 1 part by mass of the carbonate. The content is preferably 1 part by mass or less, more preferably 0.7 part by mass or less, and even more preferably 0.5 part by mass or less. Within the above range, carbonic acid can be generated such a degree that the gel film disintegrates in the digestive tract.

The content of the acid substance relative to 1 part by mass of the carbonate may be 0.1 to 1 part by mass,0.1 to 0.7 part by mass, 0.1 to 0.5 part by mass, 0.3 to 1 part by mass, 0.3 to 0.7 part by mass, 0.3 to 0.5 part by mass, 0.45 to 1 part by mass, 0.45 to 0.7 part by mass, or 0.45 to 0.5 part by mass, for example.

### (2) Second embodiment

### Packaged oral solid composition

The packaged oral solid composition in the second embodiment of the present invention is an oral solid composition comprising a gel film containing a polymer gelable on contact with water and comprising an oral solid preparation, and the oral solid composition is hermetically packed or is tightly packed together with a desiccant. The oral solid composition can be an oral solid composition in which an oral solid preparation is coated with a film containing a polymer gelable on contact with water.

### Gel film

The type and the concentration of the polymer gelable on contact with water, the type and the concentration of the containable excipient, the film thickness of the gel film, and the like are substantially the same as in the first embodiment (where the gel film in the second embodiment corresponds to the rapidly disintegrating gel film in the first embodiment).

The gel film may further contain a carbonate and an acidic substance. In the condition, after swallowing, the water-soluble polymer is dissolved by contact with water, and the carbonate and the acidic substance react with each other to cause foaming. Accordingly, the gel film disappears in the gastrointestinal tract, and the oral solid preparation coated with the gel film rapidly elutes an active ingredient. The gel film may be a single layer containing the polymer gelable on contact with water, the carbonate, and the acidic substance. The types, the concentrations, and the amounts of the carbonate and the acidic substance, the layer structure, and the like are substantially the same as in the first embodiment. The gel film is a rapidly disintegrating gel film of a first aspect in the second embodiment.

The rapidly disintegrating gel film of the first aspect is substantially the same as the rapidly disintegrating gel film in the first embodiment of the present invention. Hence, the oral solid composition comprising the rapidly disintegrating gel film of the first aspect in the second embodiment is a composition in which the oral solid composition in the first embodiment is hermetically packed or is tightly packed together with a desiccant.

The gel film may include multiple layers. For example, the gel film may be a multilayer gel film comprising a layer containing a polymer gelable on contact with water and a carbonate and a layer containing a polymer gelable on contact with water and an acidic substance (a rapidly disintegrating gel film of a second aspect in the second embodiment). The types, the concentrations, and the amounts of the carbonate and the acidic substance, the layer structure, and the like are substantially the same as in the first embodiment.

A gel film containing a carbonate or an acidic substance may be used as one layer of a rapidly disintegrating gel film having a multilayer structure (a rapidly disintegrating gel film of a third aspect in the second embodiment). For example, the rapidly disintegrating gel film may be a film comprising an undercoat containing a water-soluble polymer and a gel coat containing a polymer gelable on contact with water in this order. In the film, the undercoat contains a carbonate, and the gel coat contains an acidic substance. Alternatively, the undercoat contains an acidic substance, and the gel coat contains a carbonate.

Between the undercoat and the gel coat, a separation coat containing a water-soluble polymer may be provided. This can further effectively suppress foaming by contact between a carbonate and an acidic substance before taking.

Examples of the water-soluble polymer include those exemplified as the water-soluble coating agent (water-soluble polymer) containable in the gel film, and a polymer gelable on contact with water may also be used.

In the rapidly disintegrating gel film of the third aspect, the concentration of the carbonate is preferably 1% by mass or more, more preferably 3% by mass or more, even more preferably 5% by mass or more relative to the total amount of the rapidly disintegrating gel film. Within the above range, carbonic acid can be generated such a degree that the gel film disintegrates in the digestive tract. The concentration of the carbonate is preferably 25% by mass or less, more preferably 20% by mass or less, even more preferably 15% by mass or less relative to the total amount of the rapidly disintegrating gel film. Within the above range, generation of carbonic acid and subsequent disintegration of the gel film before dosing an oral solid composition having the gel film are suppressed.

The concentration of the carbonate relative to the total amount of the rapidly disintegrating gel film of the third aspect can be 1 to 25 % by mass, 1 to 20 % by mass, 1 to 15 % by mass, 3 to 25 % by mass, 3 to 20 % by mass, 3 to 15 % by mass, 5 to 25 % by mass, 5 to 20 % by mass, or 5 to 15 % by mass, for example.

In the rapidly disintegrating gel film of the third aspect, the concentration of the acid substance is preferably 0.1% by mass or more, more preferably 1% by mass or more, even more preferably 3% by mass or more relative to the total amount of the rapidly disintegrating gel film. Within the above range, carbonic acid can be generated such a degree that the gel film disintegrates in the digestive tract.

The concentration of the acid substance is preferably 20% by mass or less, more preferably 15% by mass or less, even more preferably 10% by mass or less relative to the total amount of the rapidly disintegrating gel film. Within the above range, generation of carbonic acid and subsequent disintegration of the gel film before dosing an oral solid composition having the gel film are suppressed.

The concentration of the acid substance relative to the total amount of the rapidly disintegrating gel film of the third aspect may be 0.1 to 20 % by mass, 0.1 to 15 % by mass, 0.1 to 10 % by mass, 1 to 20 % by mass, 1 to 15 % by mass, 1 to 10 % by mass, 3 to 20% by mass, 3 to 15 % by mass, or 3 to 10 % by mass, for example.

In the rapidly disintegrating gel film of the third aspect, the content of the acid substance is preferably 0.1 part by mass or more, more preferably 0.3 part by mass or more, even more preferably 0.45 part by mass or more relative to 1 part by mass of the carbonate. The content is preferably 1 part by mass or less, more preferably 0.7 part by mass or less, and even more preferably 0.5 part by mass or less. Within the above range, carbonic acid can be generated such a degree that the gel film disintegrates in the digestive tract.

In the rapidly disintegrating gel film of the third aspect, the content of the acid substance relative to 1 part by mass of the carbonate may be 0.1 to 1 part by mass, 0.1 to 0.7 part by mass, 0.1 to 0.5 part by mass, 0.3 to 1 part by mass, 0.3 to 0.7 part by mass, 0.3 to 0.5 part by mass, 0.45 to 1 part by mass, 0.45 to 0.7 part by mass, or 0.45 to 0.5 part by mass, for example.

### Oral solid composition

In the second embodiment of the present invention, the oral solid composition is a composition comprising the above-mentioned gel film in the second embodiment and an oral solid preparation. The oral solid composition can be a composition in which an oral solid preparation is coated with the gel film in the second embodiment.

The dosage form, the active ingredient, and the size of the oral solid preparation, the ratio of the mass of the gel film relative to the total mass of the oral solid composition indicating the thickness of the gel film, the machine usable to form the gel film, and the like are substantially the same as in the first embodiment (where the gel film in the second embodiment corresponds to the rapidly disintegrating gel film in the first embodiment). The oral solid composition can be a pharmaceutical composition, a quasi-drug composition, or a food composition as with the first embodiment.

The oral solid composition may comprise a plurality of gel films having the same formulation or different formulations. When a plurality of gel films are included, the total thickness of them can be within the above range. Between the gel film and the oral solid preparation and/or on the outside of the gel film, one or more water-soluble polymer-containing layers may be provided. The water-soluble polymer-containing layer may contain any of a polymer gelable on contact with water and a polymer not gelable even on contact with water.

### Package

A hermetic package is the package that prevents gas, liquid, and solid substances from entering. For example, a plastic bottle, a bag molded from a plastic sheet, a bag molded from a sheet comprising an aluminum layer (such as an aluminum pillow), or the like is used to hermetically pack the composition. The sheet comprising an aluminum layer may be a sheet composed of only an aluminum layer or a sheet in which one or more aluminum layers and one or more plastic layers are laminated. A sheet in which an aluminum layer and a plastic layer are laminated includes a sheet in which an aluminum sheet and a plastic sheet are laminated and a sheet comprising a plastic sheet and an aluminum deposited layer.

The bottle may be an integrated bottle with no joint. To seal a bag molded from a plastic sheet or a bag molded from a sheet comprising an aluminum layer, for example, a plastic layer-containing film functioning as an adhesive, such as an aluminum-deposited PET film, is interposed between the joint faces of sheets, or welding is performed. The bag molded from a plastic sheet or the bag molded from a sheet comprising an aluminum layer may be an integrated bag with no joint. Products of pharmaceutical preparations or food supplements packed in an aluminum pillow are commercially available, and these are typically hermetically packed in the aluminum pillow. The aluminum pillow in which a commercial product is packaged is typically prepared by molding a sheet in which one or more aluminum film layers and one or more plastic layers are laminated or by molding a sheet having one or more plastic sheet layers and one or more aluminum deposited layers. The sheet comprising an aluminum layer may be a sheet composed of only an aluminum layer or a sheet in which one or more aluminum layers and one or more plastic layers are laminated. A sheet in which an aluminum layer and a plastic layer are laminated includes a sheet in which an aluminum sheet and a plastic sheet are laminated and a sheet comprising a plastic sheet and an aluminum deposited layer.

A tight package is the package that prevents liquid and solid substances from entering. For example, a plastic bottle, a bag molded from a plastic sheet, a bag molded from a sheet comprising an aluminum layer, or the like is used to tightly pack the composition. A bottle having a cap and a main body is commonly a tight container. Products of pharmaceutical preparations or food supplements packaged in a plastic bottle is commercially available, and these are typically tightly packed in the plastic bottle. Specifically preferred is a plastic bottle having a packing on the contact face between the main body and the cap. The plastic bottle wall may comprise an aluminum layer. A bag molded from a plastic sheet or a bag molded from a sheet comprising an aluminum layer can be tightly packed, for example, by joining the sheets with an adhesive.

The oral solid composition may be hermetically packed or tightly packed in a plastic bottle, a bag molded from a plastic sheet, a bag molded from a sheet comprising an aluminum layer, or the like directly. Alternatively, the oral solid composition in a PTP package or an SP package may be hermetically packed or tightly packed in a plastic bottle, a bag molded from a plastic sheet, a bag molded from a sheet including an aluminum layer, or the like.

Examples of the plastic include polyvinyl chloride resins, polyolefin resins, cyclic olefin resins, polystyrene resins, polyester resins, polycarbonate resins, acrylic resins, fluorocarbon resins, and polyvinyl alcohol resins.

The plastic bottle (the main body and the cap) or the plastic bag may include one or more types of plastics. When including two or more types of plastics, the bottle, cap, or bag may include a mixture of two or more types of plastics, may include laminated layers containing different types of plastics, or may partially include different types of plastics.

Examples of the polyvinyl chloride resin include polyvinyl chloride (vinyl chloride homopolymers), vinyl chloride/vinyl acetate copolymers, vinyl chloride/(meth)acrylic acid copolymers, vinyl chloride/methyl (meth)acrylate copolymers, vinyl chloride/ethyl (meth)acrylate copolymers, vinyl chloride/ethylene copolymers, vinyl chloride/propylene copolymers, and vinyl chloride/vinylidene chloride copolymers.

Examples of the polyolefin resin include polyethylenes (such as a low-density polyethylene, a high-density polyethylene, and a medium-density polyethylene), polypropylene, ethylene/propylene copolymers, ethylene/α-olefin copolymers, propylene/α-olefin copolymers, ethylene/acrylic acid copolymers, ethylene/methacrylic acid copolymers, ethylene/ethyl acrylate copolymers, ethylene/vinyl acetate copolymers, and ethylene/ethyl acrylate copolymers.

Examples of the styrene resin include styrene homopolymers, styrene/ethylene copolymers, styrene/α-olefin (such as propylene, butene, pentene, methylpentene, hexene, heptene, octene, nonene, and decene) copolymers, and styrene/acrylonitrile copolymers.

Examples of the polyester resin include polyalkylene terephthalates such as polyethylene terephthalate and polybutylene terephthalate; polyalkylene naphthalates such as polyethylene naphthalate and polybutylene naphthalate; and polycycloalkylene terephthalates such as poly (1,4-cyclohexylene dimethylene terephthalate).

The cyclic olefin resin (COP) includes a cyclic olefin copolymer (COC).

Examples of the fluorocarbon resin include polychlorotrifluoroethylene, polytetrafluoroethylene, and polychlorotetrafluoroethylene.

Examples of the polyvinyl alcohol resin include polyvinyl alcohol and ethylene/vinyl alcohol copolymers.

Of them, polyvinyl chloride resins and polyolefin resins are preferred, and a homopolymer of vinyl chloride and polypropylene are more preferred.

The plastic may contain excipients such as a plasticizer. By adding an ultraviolet absorber, an ultraviolet scattering agent, a coloring agent, or the like, discoloration of the gel film can be more effectively suppressed.

The bottle wall of the plastic bottle (for a bottle having a cap and a main body, at least the main body wall) may have a thickness of 0.5 mm or more, 1 mm or more, or 2 mm or more. Within the range, discoloration of the gel film can be suppressed. The bottle wall of the plastic bottle (for a bottle having a cap and a main body, at least the main body wall) may have a thickness of 10 mm or less. Within the range, the bottle is easily handled and is produced at low costs. The bottle wall of the plastic bottle may have a thickness of 0.5 to 10 mm, 1 to 10 mm, or 2 to 10 mm.

The plastic bottle may have any shape. For example, the plastic bottle may have a tubular shape (a cylindrical shape or a prismatic shape), and the tube portion may be bulged or dented in the center part, the lower part, or the upper part. The plastic bottle may have such a size that one or more oral solid compositions can be placed therein.

The bag molded from a plastic sheet or the bag molded from a sheet having an aluminum layer may have a thickness of 10 µm or more, 20 µm or more, or 40 µm or more. Within the range, discoloration of the gel film can be suppressed. The bag molded from a plastic sheet or the bag molded from a sheet including an aluminum layer may have a thickness of 80 µm or less. Within the range, the cost can be reduced.

For the sheet including an aluminum layer (for a laminate including an aluminum layer and a plastic layer, and for an plastic sheet on which aluminum is deposited), the aluminum layer may have a thickness of 5 µm or more, 10 µm or more, 15 µm or more, or 20 µm or more. Within the range, discoloration of the gel film can be suppressed. In this case, the aluminum layer may have a thickness of 40 µm or less. Within the range, the cost can be reduced.

The bag may have such a size that one or more oral solid compositions can be placed therein.

As described above, when tightly packed, the oral solid composition is packaged together with a desiccant. When hermetically packed, the oral solid composition can also be packaged together with a desiccant. This can further effectively suppress discoloration of the gel film. "Packaging an oral solid composition together with a desiccant" means that an oral solid composition comprising a gel film is stored together with a desiccant in the space of a single container (such as a bottle or a bag) .

Examples of the desiccant include silica gel, allophane, Siblet, zeolite, calcium chloride, and magnesium chloride. Of them, silica gel and calcium chloride are preferred. One or more types of desiccants may be used.

### (3) Third embodiment

### (3-1) Gel film

The gel film in the third embodiment of the present invention is a gel film formed by using a coating liquid comprising a polymer gelable on contact with water. The concentration of glycerol in the coating liquid is 2% by mass or less relative to the total amount of the gel film, and the polymer gelable on contact with water is partially or practically completely (especially completely) dispersed in the coating liquid.

### Glycerol

In the gel film in the third embodiment, the glycerol concentration is 2% by mass or less relative to the total amount of the gel film, and can be 1.8% by mass or less, 1.6% by mass or less, 1.4% by mass or less, 1.2% by mass or less, or 1% by mass or less. The gel film may contain substantially no glycerol (especially contain no glycerol). This can prevent mold from growing.

In the present invention, when a glycerol product to be added contains water, the glycerol concentration is the concentration of the glycerol contained in the glycerol product relative to the total amount of a gel film.

The type and the concentration of the polymer gelable on contact with water are the same as in the rapidly disintegrating gel film in the first embodiment. When the polymer concentration is within the range, the produced film is easily swallowed, and mold growth or stickiness on the surface of the gel film can be suppressed.

The type and the concentration or amount of the containable excipient and the film thickness of the gel film are the same as those of the rapidly disintegrating gel film in the first embodiment (where the gel film in the third embodiment corresponds to the rapidly disintegrating gel film in the first embodiment).

The gel film can be a rapidly disintegrating gel film of a single layer containing a carbonate and an acidic substance of a first aspect, a rapidly disintegrating gel film including multiple layers containing a carbonate and an acidic substance of a second aspect, or a rapidly disintegrating gel film containing a carbonate or an acidic substance of a third aspect, as with the second embodiment. The types and the concentrations or amounts of the carbonate and the acidic substance, the layer structure, and the like are the same as in the second embodiment. However, in the rapidly disintegrating gel film in the third embodiment, the glycerol concentration is 2% by mass or less relative to the total amount of the gel film, and the gel film is formed by using a coating liquid in which the polymer gelable on contact with water is partially or practically completely dispersed.

### (3-2) Coating liquid

The third embodiment of the present invention encompasses a coating liquid containing a polymer gelable on contact with water, in which the concentration of glycerol is 2% by mass or less relative to the total amount of the solid content in the coating liquid, and the polymer gelable on contact with water is partially, practically completely, or completely dispersed. The coating liquid can be for coating an oral solid preparation. The gel film in the third embodiment may be formed by using the coating liquid.

The coating liquid contains the polymer gelable on contact with water in a partially dispersed state, a practically completely dispersed state, or a completely dispersed state. Hence, a part, practically all, or all of the solvent contained in the coating liquid may be a solvent in which the polymer gelable on contact with water is not dissolved. Specifically, practically all, particularly all of the solvent contained in the coating liquid is preferably a solvent in which the polymer gelable on contact with water is not dissolved. Examples of such a solvent include polar solvents other than water, such as lower alcohols (including methanol, ethanol, and isopropanol), and nonpolar solvents such as acetone. Of them, polar solvents other than water are preferred, and ethanol is more preferred.

Even when the solvent contained in the coating liquid is water, the polymer gelable on contact with water may be contained in a partially dispersed state due to supersaturation.

The coating liquid in the third embodiment may contain one or more excipients exemplified in the first embodiment.

### (3-3) Oral solid composition

The oral solid composition of the present invention is a composition comprising the above-described gel film in the third embodiment and an oral solid preparation. The oral solid composition of the present invention can be a composition in which an oral solid preparation is coated with the gel film in the third embodiment.

The dosage form, the active ingredient, and the size of the oral solid preparation, the ratio of the mass of the gel film relative to the total mass of the oral solid composition indicating the thickness of the gel film, the machine usable to form the gel film, and the like are substantially the same as in the first embodiment (where the gel film in the third embodiment corresponds to the rapidly disintegrating gel film in the first embodiment). The oral solid composition can be a pharmaceutical composition, a quasi-drug composition, or a food composition as with the first embodiment.

The oral solid composition may comprise a plurality of gel films having the same formulation or different formulations. When a plurality of gel films are included, the total thickness of them can be within the above range. Between the gel film and the oral solid preparation and/or on the outside of the gel film, one or more water-soluble polymer-containing layers may be provided. The water-soluble polymer-containing layer may contain any of a polymer gelable on contact with water and a polymer not gelable even on contact with water.

The oral solid composition in the third embodiment can be produced as follows: by coating an oral solid preparation with a coating liquid in which a polymer gelable on contact with water is dispersed, a gel film is formed such that the glycerol concentration is 2% by mass or less relative to the total amount of the gel film. The third embodiment thus encompasses a method for producing an oral solid composition, and the method comprises a step of coating an oral solid preparation with the coating liquid in the third embodiment. When the coating liquid in the third embodiment is used to coat an oral solid preparation, a gel film in which the glycerol concentration is 2% by mass or less relative to the total amount of the gel film is formed.

### (4) Fourth embodiment

### (4-1) Gel film

The gel film in the fourth embodiment of the present invention is a gel film containing a polymer gelable on contact with water, containing titanium oxide (TiO₂) at 20% by mass or less relative to the total amount of the gel film, and having a surface that is marked without engraving or any coloring agent.

The concentration of the titanium oxide is 20% by mass or less relative to the total amount of the gel film. The concentration can be 15% by mass or less, 10% by mass or less, 8% by mass or less, 6% by mass or less, or 4% by mass or less. The concentration of the titanium oxide is more than 0% by mass relative to the total amount of the gel film. The concentration can be 0.1% by mass or more, 0.2% by mass or more, 0.3% by mass or more, 0.4% by mass or more, 0.5% by mass or more, 0.6% by mass or more, 0.8% by mass or more, 1% by mass or more, 1.5% by mass or more, or 2% by mass or more. Within the above range, characters or the like can be identifiably displayed by ultraviolet laser irradiation.

The concentration of the titanium oxide relative to the total amount of the gel film is more than 0% by mass and 20% by mass or less, more than 0% by mass and 15% by mass or less, more than 0% by mass and 10% by mass or less, more than 0% by mass and 8% by mass or less, more than 0% by mass and 6% by mass or less, more than 0% by mass and 4% by mass or less, 0.1 to 20 % by mass, 0.1 to 15 % by mass, 0.1 to 10 % by mass, 0.1 to 8 % by mass, 0.1 to 6 % by mass, 0.1 to 4 % by mass, 0.2 to 20 % by mass, 0.2 to 15 % by mass, 0.2 to 10 % by mass, 0.2 to 8 % by mass, 0.2 to 6 % by mass, 0.2 to 4 % by mass, 0.3 to 20 % by mass, 0.3 to 15 % by mass, 0.3 to 10 % by mass, 0.3 to 8 % by mass, 0.3 to 6 % by mass, 0.3 to 4 % by mass, 0.4 to 20 % by mass, 0.4 to 15 % by mass, 0.4 to 10 % by mass, 0.4 to 8 % by mass, 0.4 to 6 % by mass, 0.4 to 4 % by mass, 0.5 to 20 % by mass, 0.5 to 15 % by mass, 0.5 to 10 % by mass, 0.5 to 8 % by mass, 0.5 to 6 % by mass, 0.5 to 4 % by mass, 0.6 to 20 % by mass, 0.6 to 15 % by mass, 0.6 to 10 % by mass, 0.6 to 8 % by mass, 0.6 to 6 % by mass, 0.6 to 4 % by mass, 0.8 to 20 % by mass, 0.8 to 15 % by mass, 0.8 to 10 % by mass, 0.8 to 8 % by mass, 0.8 to 6 % by mass, 0.8 to 4 % by mass, 1 to 20 % by mass, 1 to 15 % by mass, 1 to 10 % by mass, 1 to 8 % by mass, 1 to 6 % by mass, 1 to 4 % by mass, 1.5 to 20 % by mass, 1.5 to 15 % by mass, 1.5 to 10 % by mass, 1.5 to 8 % by mass, 1.5 to 6 % by mass, 1.5 to 4 % by mass, 2 to 20 % by mass, 2 to 15 % by mass, 2 to 10 % by mass, 2 to 8 % by mass, 2 to 6 % by mass, or 2 to 4 % by mass, for example.

The type and the concentration of the polymer gelable on contact with water, the type and the concentration of the containable excipient, and the like are the same as in the first embodiment (where the gel film in the fourth embodiment corresponds to the rapidly disintegrating gel film in the first embodiment). The gel film in the fourth embodiment may not contain any coloring agent other than titanium oxide.

The film thickness of the gel film is the same as that of the rapidly disintegrating gel film in the first embodiment. Within the range, the gel film can be clearly marked, and an active ingredient in an oral solid preparation can be rapidly eluted.

### Marking

"Marking" means displaying characters, numeric characters, symbols, or the like.

On the gel film in the fourth embodiment, the gel film surface is not marked by engraving that changes the surface shape of an oral solid preparation, or the gel film surface is not marked by using a coloring agent such as an ink or is not marked by printing. The gel film in the fourth embodiment can be marked by ultraviolet laser irradiation.

The gel film can be a rapidly disintegrating gel film of a single layer containing titanium oxide at 20% by mass or less, a carbonate, and an acidic substance of a first aspect, a rapidly disintegrating gel film including multiple layers containing titanium oxide at 20% by mass or less, a carbonate, and an acidic substance of a second aspect, or a rapidly disintegrating gel film containing titanium oxide at 20% by mass or less and a carbonate or an acidic substance of a third aspect, as with the second embodiment. The types and the concentrations or amounts of the carbonate and the acidic substance, the layer structure, and the like are the same as in the second embodiment.

### (4-2) Coating composition

The fourth embodiment of the present invention provides a coating composition for forming a gel film having a surface with marking, and the coating composition comprises a polymer gelable on contact with water and comprises titanium oxide at 20% by mass or less relative to the total amount of the coating composition. The composition is solid, and thus the concentration of titanium oxide is the concentration relative to the total amount of the solid contents. The coating composition can be for coating an oral solid preparation.

By coating an oral solid preparation with a coating liquid prepared by mixing the coating composition with a solvent, an oral solid composition coated with a gel film can be produced. Examples of the solvent include polar solvents such as lower alcohols including water and ethanol and nonpolar solvents such as acetone, and a person skilled in the art can select a suitable solvent.

The coating liquid preferably contains the polymer gelable on contact with water in a partially dispersed state, a practically completely dispersed state, or a completely dispersed state. Hence, a part, practically all, or all of the solvent contained in the coating liquid is preferably a solvent in which the polymer gelable on contact with water is not dissolved. Specifically, practically all, particularly all of the solvent contained in the coating liquid is more preferably a solvent in which the polymer gelable on contact with water is not dissolved. Examples of such a solvent include polar solvents other than water, such as lower alcohols (including methanol, ethanol, and isopropanol), and nonpolar solvents such as acetone. Of them, polar solvents other than water are preferred, and ethanol is more preferred. When the solvent contained in the coating liquid is water, the polymer gelable on contact with water may be contained in a partially dispersed state due to supersaturation.

The coating composition in the fourth embodiment may contain one or more excipients exemplified in the first embodiment.

Hence, the gel film in the fourth embodiment is preferably a film formed by using a coating liquid in which the polymer gelable on contact with water is partially or completely dispersed.

### (3) Oral solid composition

The oral solid composition in the fourth embodiment of the present invention is a composition comprising the above-described gel film in the fourth embodiment and an oral solid preparation. The oral solid composition in the fourth embodiment can be a composition in which an oral solid preparation is coated with the gel film in the fourth embodiment.

The dosage form, the active ingredient, and the size of the oral solid preparation, the machine used to form the gel film, and the like are the same as in the first embodiment (where the gel film in the fourth embodiment corresponds to the rapidly disintegrating gel film in the first embodiment). The ratio of the mass of the gel film relative to the total mass of the oral solid composition indicating the thickness of the gel film is the same as in the first embodiment (where the gel film in the fourth embodiment corresponds to the rapidly disintegrating gel film in the first embodiment). Within the range, the gel film surface can be clearly marked, and an active ingredient in the oral solid preparation can be rapidly eluted. The oral solid composition can be a pharmaceutical composition, a quasi-drug composition, or a food composition as with the first embodiment.

The oral solid composition may comprise a plurality of gel films having the same formulation or different formulations. When a plurality of gel films are included, the total thickness can be within the above range. Between the gel film and the oral solid preparation, one or more water-soluble polymer-containing layers may be provided. The water-soluble polymer-containing layer may contain any of a polymer gelable on contact with water and a polymer not gelable even on contact with water.

### (4-4) Method for producing gel film or oral solid composition

The gel film in the fourth embodiment of the present invention can be produced by a method comprising a step of irradiating the surface of a gel film containing a polymer gelable on contact with water and containing titanium oxide at 20% by mass or less relative to the total amount of the gel film, with ultraviolet laser light. By ultraviolet laser irradiation, characters, numeric characters, symbols, or the like may be displayed. Typically, an oral solid preparation is coated with a gel film, and then the gel film surface is irradiated with ultraviolet laser light.

Hence, the oral solid composition in the fourth embodiment can be produced by a method comprising a step of coating an oral solid preparation with a gel film containing a polymer gelable on contact with water and containing titanium oxide at 20% by mass or less relative to the total amount of the gel film and a step of irradiating the surface of the gel film with ultraviolet laser light.

The machine usable to form the gel film is the same as in the first embodiment (where the gel film in the fourth embodiment corresponds to the rapidly disintegrating gel film in the first embodiment).

The wave length of the ultraviolet laser light can be 200nm or more, 250nm or more, 300nm or more, or 350nm or more. The wave length of the ultraviolet laser light can be 550nm or less, 500nm or less, 450nm or less, or 400nm or less. Within the above range, a characters, numeric characters, symbols, or the like can be clearly displayed.

The wave length of the ultraviolet laser light may be 200 to 550nm, 200 to 500nm, 200 to 450nm, 200 to 400nm, 250 to 550nm, 250 to 500nm, 250 to 450nm, 250 to 400nm, 300 to 550nm, 300 to 500nm, 300 to 450nm, 300 to 400nm, 350 to 550nm, 350 to 500nm, 350 to 450nm, or 350 to 400nm, for example.

The output power of the ultraviolet laser light can be 0.1W or more, 1W or more, 5W or more, or 10W or more. The output power can be 50W or less, 40W or less, 30W or less, or 20W or less. Within the above range, characters, numeric characters, symbols, or the like can be clearly displayed.

The output power of the ultraviolet laser light may be 0.1 to 50 W, 0.1 to 40 W, 0.1 to 30W, 0.1 to 20 W, 1 to 50 W, 1 to 40 W, 1 to 30 W, 1 to 20 W, 5 to 50 W, 5 to 40 W, 5 to 30 W, 5 to 20 W, 10 to 50 W, 10 to 40 W, 10 to 30 W, or 10 to 20 W, for example.

### EXAMPLES

The present invention will next be described in further detail with reference to Examples, but the present invention is not limited to them.

### (1) First embodiment

### (1-1) Evaluation of disintegration, solubility, and slidability of gel film

### Production of uncoated tablets containing acetaminophen

In a container-rotating mixer (Tumbler Mixer TM-15S, Showa Kagakukikai), 0.4 kg of acetaminophen, 2.64 kg of D-mannitol (Granutol S, Freund Corporation), 0.8 kg of microcrystalline cellulose (Ceolus PH-102, Asahi Kasei), 120 g of croscarmellose sodium (Ac-Di-Sol, Dupont), and 40 g of magnesium stearate (plant-based magnesium stearate, Taihei Chemical Industrial) were placed and mixed. The mixture was tableted by using a rotary tableting machine (VIRGO24, Kikusui Seisakusho) to give uncoated tablets having a tablet weight of 400 mg and a tablet thickness of 4.7 mm.

Contents of ingredients per uncoated tablet are shown in table 1-1.

**Table 1-1**

| Ingredient | Content(mg) |
|---|---|
| Acetaminophen | 40.00 |
| D-Mannitol | 264.00 |
| Microcrystalline cellulose | 80.00 |
| Croscarmellose sodium | 12.00 |
| Magnesium stearate | 4.00 |

### Production of tablets coated with rapidly disintegrating gel film

In absolute ethanol, xanthan gum (SAN-ACE S, San-Ei Gen F.F.I), talc (ML-115, FUJI TALC INDUSTRIAL), hydroxypropyl cellulose (HPC-SL, Nippon Soda), glycerol (concentrated glycerin, Sakamoto Yakuhin Kogyo), sodium hydrogen carbonate (sodium hydrogen carbonate, ASAHI GLASS), and citric acid hydrate (citric acid hydrate, Showa Kako) were dissolved or dispersed to give a gel coating liquid. The prepared uncoated tablets having a weight of 400 mg and containing acetaminophen were subjected to film coating by using a coating machine (HICOATER, Freund Corporation) so as to have a predetermined weight, giving tablets coated with a gel film.

The formulae of the gel films of the tablets prepared in Examples 1-1 to 1-7 and Comparative Examples 1-1 to 1-3 are shown in Table 1-2.

**Table 1-2**

| Unit: mg per tablet (Parenthesized value is % by mass relative to the total amount of the film) | | | | | | | | | | |
|---|---|---|---|---|---|---|---|---|---|---|
| | Example 1-1 | Example 1-2 | Example 1-3 | Example 1-4 | Example 1-5 | Example 1-6 | Example 1-7 | Comparative Example 1-1 | Comparative Example 1-2 | Comparative Example 1-3 |
| Xanthan gum | 6.94 | 3.47 | 3.47 | 1.16 | 3.47 | 3.47 | 6.94 | 6.94 | - | - |
| Talc | 0.99 | 0.99 | 0.99 | 0.99 | 0.99 | 0.99 | 1.98 | 0.99 | - | 0.99 |
| Hydroxypropyl cellulose | 3.96 | 3.96 | 3.96 | 3.96 | 3.96 | 3.96 | 7.92 | 3.96 | - | 7.43 |
| Glycerol | 0.89 | 0.89 | 0.89 | 0.89 | 0.89 | 0.89 | 1.78 | 0.89 | - | 0.89 |
| Sodium hydrogen carbonate | 5.34 (26.0%) | 2.67 (20.3%) | 1.00 (9.29%) | 2.67 (24.6%) | - | 2.67 (20.3%) | 2.67 (11.9%) | - | - | 2.67 (20.3%) |
| Potassium hydrogen carbonate | - | - | - | - | 2.67 (20.3%) | - | - | - | - | - |
| Citric acid hydrate | 2.38 (16.6%) | 1.19 (9.04%) | 0.45 (4.18%) | 1.19 (11.0%) | 1.19 (9.04%) | - | 1.19 (5.29%) | 2.38 (15.7%) | - | 1.19 (9.04%) |
| Ascorbic acid | - | - | - | - | - | 1.19 (9.04%) | - | - | - | - |
| Mass ratio of film relative to the total amount of the tablet | 4.88% | 3.19% | 2.62% | 2.64% | 3.19% | 3.19% | 5.32% | 3.65% | - | 3.19% |
| Slidability | ○ | ○ | ○ | ○ | ○ | ○ | ○ | ○ | × | × |
| Dissolution rate at 15 minutes after start of test | 90.2% | 88.5% | 94.1% | 97.1% | 92.0% | 87.9% | 88.0% | 83.6% | 98.8% | 97.9% |

### Test 1 (Evaluation of gel film disintegration and solubility)

Dissolution test was performed to estimate disintegration of the gel film and solubility regarding tablets obtained in Examples 1-1 to 1-7 and comparative examples 1-1 to 1-3.

### (Dissolution test)

The dissolution test was performed in accordance with the second method of the dissolution test described in the Japanese Pharmacopoeia. Nine hundred mL of water was used as a test solution. The test solution temperature was set at 37°C, paddle rotation rate was set at 50 rpm (NTR-6400AC, TOYAMA SANGYO CO., LTD.). At testing times of 5, 10, 15 minutes, an eluate was sampled, and quantitated the amount of eluted acetaminophen by high performance liquid chromatography to estimate elution behavior.

### <HPLC conditions>

Column: InertSustain C18 (4.6×150mm)
Column temperature: 40°C
Developing solvent: diluted acetic acid (1→1000) /acetonitrile mixture (17:3)
Flow rate of developing solvent: 1.0mL/minute
Detector: Absorbance detector of ultraviolet and visible lights

The dissolution rates of acetaminophen after 15 minutes are shown in Table 1-2. The tablets having a gel film containing an acidic substance and a carbonate in Examples 1-1 to 1-7 had a dissolution rate of 88% or more. The uncoated tablet having no film in Comparative Example 1-2 had a very good dissolution rate of 98.8%. The tablet having a film containing only a water-soluble polymer not gelable even on contact with water in Comparative Example 1-3 had a very good dissolution rate of 97.9%. In contrast, the tablet having a gel film containing no carbonate in Comparative Example 1-1 had a low dissolution rate of 83.6%. It is revealed that in the tablet in Comparative Example 1-1, the gel film disintegrated slowly, and this interfered with elution of the active ingredient.

According to "Guideline for Bioequivalence Studies of Formulation Changes for Oral Solid Preparations", when a standard preparation elutes 85% or more of an active ingredient within 15 minutes, a tested preparation that elutes 85% or more of an active ingredient within 15 minutes is biologically equivalent to the standard preparation (March 19, 2020, Notification 0319 No. 1, Appendix 3 issued by the Director of Pharmaceutical Safety and Environmental Health Bureau). The preparation in Comparative Example 1-3 (dissolution rate: 97.9%) is generally the standard preparation, and thus a preparation having a dissolution rate of 85% or more is biologically equivalent to the standard preparation.

### Test 2 (Evaluation of gel film slidability)

The slidability of each tablet prepared in Examples 1-1 to 1-7 and Comparative Examples 1-1 to 1-3 was evaluated on an acrylic plate.

An acrylic plate was placed at an angle of 20° to the horizontal plane. The bottom face of a tablet was slightly wetted with a damp sponge, and the tablet was placed on the upper part of the acrylic plate. From a position higher than the placed tablet (from the upstream side), about 50 mL of water was allowed to slowly flow, and whether the tablet flowed was evaluated. A tablet flowing with running water was evaluated as o, and a tablet not flowing was evaluated as ×.

The results are shown together in Table 1-2. All the tablets coated with a gel film prepared in Examples 1-1 to 1-7 and Comparative Example 1-1 rapidly slid down the acrylic plate, and the gel film was found to function. In contrast, the tablet with no film in Comparative Example 1-2 and the tablet with the film containing only a water-soluble polymer not gelable even on contact with water in Comparative Example 1-3 strongly adhered to the acrylic plate and were difficult to slide down. It is revealed that a tablet having a film gelable on contact with water has higher slidability.

### (1-2) Evaluation of gel film stability

### Production of tablets with gel film

In a container-rotating mixer (Tumbler Mixer TM-15S, Showa Kagakukikai), 200 g of acetaminophen, 1.32 kg of D-mannitol (Granutol S, Freund Corporation), 400 g of crystalline cellulose (Ceolus PH-102, Asahi Kasei), 60 g of croscarmellose sodium (Ac-Di-Sol, Dupont), and 20 g of magnesium stearate (plant-based, Taihei Chemical Industrial) were placed and mixed. The mixture was tableted by using a rotary tableting machine (VIRGO24, Kikusui Seisakusho) to give uncoated tablets having a tablet weight of 400 mg and a tablet thickness of 4.7 mm.

The uncoated tablet was coated with a single-layer gel film containing 6.94 mg of xanthan gum, 0.99 mg of talc, 3.96 mg of hydroxypropyl cellulose, 0.89 mg of glycerol (concentrated glycerin), 5.34 mg of sodium hydrogen carbonate, and 2.38 mg of citric acid hydrate.

Separately, the uncoated tablet was coated with an undercoat containing 5.33 mg of hypromellose and 2.67 mg of sodium hydrogen carbonate and further coated with a gel coat containing 6.94 mg of xanthan gum, 1.98 mg of talc, 7.92 mg of hydroxypropyl cellulose, 1.78 mg of glycerol (concentrated glycerin), and 1.19 mg of citric acid hydrate, giving a tablet with a gel film having a two-layer structure.

### Dissolution test

Tablets were preserved under the conditions of 40°C and 75%RH for 6 months.

Dissolution test was performed to evaluate disintegration of gel films and solubility regarding tablets before and after the preservation.

The dissolution test was performed in accordance with the second method of the dissolution test described in the Japanese Pharmacopoeia. Nine hundred mL of water was used as a test solution. The test solution temperature was set at 37°C, paddle rotation rate was set at 50 rpm (NTR-6100AC , TOYAMA SANGYO CO., LTD.). At testing times of 5, 10, 15 minutes, an eluate was sampled, and quantitated the amount of eluted acetaminophen by high performance liquid chromatography to estimate elution behavior.

### <HPLC conditions>

Column: InertSustain C18 (4.6×150mm)
Column temperature: 40°C
Developing solvent: diluted acetic acid (1_{→}1000) /acetonitrile mixture (17:3)
Flow rate of developing solvent: 1.0mL/minute
Detector: Absorbance detector of ultraviolet and visible lights

The dissolution rate of acetaminophen from the tablets with the single-layer gel film 15 minutes after the start of the test was 90.2% before storage and was 97.5% after storage for 6 months. Even when the tablets were stored, the gel film did not substantially change or disintegrate. The dissolution rate of acetaminophen from the tablets with the gel film having a two-layer structure 15 minutes after the start of the test was 96.9% before storage and was 96.1% after storage for 6 months. Even when the tablets were stored, the gel film did not change or disintegrate. The single-layer gel film contains sodium hydrogen carbonate and citric acid hydrate in the film, and is revealed to be stable as with the gel film having a two-layer structure in which sodium hydrogen carbonate and citric acid hydrate are contained in separate layers.

### (1-3) Bioequivalence study

### Production of tablets

Uncoated tablets having a diameter of 13.0 mm, a thickness of 6 mm, and a weight of 250 mg were produced in a common procedure. The uncoated tablets contained 40% by mass of an active ingredient, 43% of microcrystalline cellulose, 2.4% of hydroxypropyl cellulose, 10% of low-substituted hydroxypropyl cellulose, 0.5% by mass of light anhydrous silicic acid, 3.1% by mass of crospovidone, and 1% by mass of magnesium stearate.

### (Coating with gel film)

In absolute ethanol, xanthan gum (SAN-ACE S, San-Ei Gen F.F.I), talc (ML-115, FUJI TALC INDUSTRIAL), hydroxypropyl cellulose (HPC-SL, Nippon Soda), and titanium oxide (KRONOS 1171, Kronos Worldwide, Inc.) were dissolved or dispersed to give a gel coating liquid.

The uncoated tablets were subjected to film coating by using a coating machine (HICOATER, Freund Corporation) such that the mass ratio of the gel film was 3.8% by mass relative to the uncoated tablet, giving tablets coated with a gel film.

### (Common film coating)

In purified water, OPADRYII 85F420044 Yellow was dissolved and dispersed to give a coating liquid.

The uncoated tablets were subjected to film coating by using a coating machine (HICOATER, Freund Corporation) such that the mass ratio of the film was 3.8% by mass relative to the uncoated tablet, giving tablets coated with a film.

### Bioequivalence study

Bioequivalence study was performed on 15 healthy adult males for common film-coated tablets and gel film-coated tablets, by single oral dose crossover method with a washout period. The plasma concentrations of the active ingredient were measured for 48 hours after administration, and pharmacokinetic parameters, AUC (area under the (blood plasma concentration-time) curve) and Cmax, were compared.

A graph of changes in average plasma concentration is shown in Figure 1. From the AUC and the Cmax of the common film-coated tablets and those of the gel film-coated tablets, the preparations were ascertained to be biologically equivalent.

The result reveals that the gel film does not affect the changes over time in plasma concentration of an active ingredient.

### (2) Second embodiment

### Evaluation of color stability

### Production of tablets coated with gel film

In a container-rotating mixer (Tumbler Mixer TM-15S, Showa Kagakukikai), 3.384 kg of lactose hydrate (Dilactose F, Freund Corporation), 180 g of hydroxypropyl cellulose (HPC-L-FP, Nippon Soda), and 36 g of magnesium stearate (plant-based, Taihei Chemical Industrial) were placed and mixed. The mixture was tableted by using a rotary tableting machine (VIRGO24, Kikusui Seisakusho) to give uncoated tablets having a tablet weight of 180 mg and a tablet thickness of 2.9 mm.

In absolute ethanol, xanthan gum (SAN-ACE S, San-Ei Gen F.F.I), talc (ML-115, FUJI TALC INDUSTRIAL), hydroxypropyl cellulose (HPC-SL, Nippon Soda), and titanium oxide (KRONOS 1171, Kronos Worldwide, Inc.) were dissolved or dispersed to give a gel coating liquid. The xanthan gum was dispersed in the gel coating liquid.

The uncoated tablets were subjected to film coating by using a coating machine (HICOATER, Freund Corporation) such that the mass ratio of the film was 3% by mass relative to the uncoated tablet, giving tablets coated with a gel film.

The formula of the gel film is shown in Table 2-1. The amount of each ingredient in the gel film shown in Table 2-1 is the same as the amount of the corresponding ingredient contained in one coated tablet of the solid composition.

**Table 2-1**

| | |
|---|---|
| Xanthan gum | 1.47 mg |
| Talc | 0.42 mg |
| Hydroxypropyl cellulose | 1.70 mg |
| Sodium hydrogen carbonate | 1.13 mg |
| Citric acid hydrate | 0.51 mg |
| Titanium oxide | 0.07 mg |

The above tablets were packaged in packaging materials shown in Table 2-2 and were allowed to stand for 4 weeks in conditions at 55°C and 75% RH.

The aluminum pillow (aluminate zip bag, Meiwa Pax) used in (1) to (8), (11), and (12) in Table 2-2 is composed of an aluminum/polyethylene laminated film (PET#12/AC/PE15 µm/AL#9/AC/PE40 µm), and the joint portion is sealed with an aluminum deposited PET film. The bottle comprises a polyethylene main body having a thickness of 2 mm and a polypropylene cap, and between the cap and the main body, a packing is interposed. The PTP package is composed of a plastic concave portion shown below and an aluminum sheet. For tablets packed together with a desiccant, calcium chloride was used as the desiccant.

Before and after standing for 4 weeks, the color on the tablet surface was determined by a spectrocolorimeter (SE 7700, Nippon Denshoku Industries), and the color difference (ΔE) between before and after standing was calculated.

**Table 2-2**

| | ΔE |
|---|---|
| (1) PTP package (PVC) +aluminum pillow | 4.13 |
| (2) PTP package (PP) + aluminum pillow | 3.43 |
| (3) PTP package (PVC/PVDC) +aluminum pillow | 4.77 |
| (4) PTP package (PVC/PCTFE) + aluminum pillow | 3.65 |
| (5) PTP package (PVC)+ aluminum pillow + desiccant | 1.75 |
| (6) PTP package (PP)+ aluminum pillow + desiccant | 1.61 |
| (7) PTP package (PVC/PVDC) +aluminum pillow + desiccant | 1.58 |
| (8) PTP package (PVC/PCTFE) +aluminum pillow + desiccant | 1.81 |
| (9) bottle (Comparative Example) | 5.03 |
| (10) bottle + desiccant | 1.49 |
| (11) aluminum pillow | 4.19 |
| (12) aluminum pillow + desiccant | 1.43 |
| (13) No package (Comparative Example) | 8.83 |

| | |
|---|---|
| PVC : Polyvinyl chloride PP : Polypropylene PVC/PVDC : Polyvinyl chloride/polyvinylidene chloride laminate PVC/PCTFE : Polyvinyl chloride/Polychloro trifluoro ethylene laminate | |

In the case where the tablet was hermetically packed in an aluminum pillow or by tightly packed together with a desiccant in a bottle, the ΔE was 5 or less (indicating that colors were substantially the same when compared with time), and discoloration was greatly suppressed. By packaging tablets together with a desiccant in a hermetic container, discoloration was markedly suppressed. Meanwhile, when tablets were tightly packed without any desiccant in a bottle ((9) in Table 2-2), the ΔE exceeded 5, and the color difference was such a degree as to sense discoloration.

### (3) Third embodiment

### Production of tablets coated with gel film

In a container-rotating mixer (Tumbler Mixer TM-15S, Showa Kagakukikai), 3.384 kg of D-mannitol (Granutol F, Freund Corporation), 180 g of hydroxypropyl cellulose (HPC-L-FP, Nippon Soda), and 36 g of magnesium stearate (plant-based, Taihei Chemical Industrial) were placed and mixed. The mixture was tableted by using a rotary tableting machine (VIRGO24, Kikusui Seisakusho) to give uncoated tablets having a tablet weight of 180 mg and a tablet thickness of 2.9 mm.

### (Examples 3-1 to 3-3)

In absolute ethanol, xanthan gum (SAN-ACE S, San-Ei Gen F.F.I), talc (ML-115, FUJI TALC INDUSTRIAL), hydroxypropyl cellulose (HPC-SL, Nippon Soda), and titanium oxide (KRONOS 1171, Kronos Worldwide, Inc.) were dissolved or dispersed to give a gel coating liquid. The xanthan gum was dispersed in the gel coating liquid.

The uncoated tablets were subjected to film coating by using a coating machine (HICOATER, Freund Corporation), giving tablets coated with a gel film.

### (Example 3-4, Comparative Examples 3-1 and 3-2)

In absolute ethanol, xanthan gum (SAN-ACE S, San-Ei Gen F.F.I), talc (ML-115, FUJI TALC INDUSTRIAL), hydroxypropyl cellulose (HPC-SL, Nippon Soda), glycerol (concentrated glycerin, Sakamoto Yakuhin Kogyo), and titanium oxide (KRONOS 1171, Kronos Worldwide, Inc.) were dissolved or dispersed to give a gel coating liquid. The xanthan gum was dispersed in the gel coating liquid. The concentration of glycerol in the concentrated glycerin was 98 to 101%.

The uncoated tablets were subjected to film coating by using a coating machine (HICOATER, Freund Corporation), giving tablets coated with a gel film.

### (Comparative Example 3-3)

Film coating was performed in the same procedure as in Example 3-1 with the same gel coating liquid as in Example 3-1 except that purified water was used in the same amount in place of absolute ethanol to prepare the gel coating liquid.

The xanthan gum was dissolved in the gel coating liquid, and the gel coating liquid was highly viscous. During coating, the gel coating liquid clogged nozzles of the coating apparatus, and the uncoated tablets were failed to be coated with a film.

The formulae of the gel films on the tablets prepared in Examples 3-1 to 3-4 and Comparative Examples 3-1 to 3-3 are shown in Table 3-1.

**Table 3-1**

| Unit: mg in the gel film of one tablet | | | | | | | |
|---|---|---|---|---|---|---|---|
| | Example3-1 | Example 3-2 | Example 3-3 | Example 3-4 | Comparative Example 3-1 | Comparative Example 3-2 | Comparative Example 3-3 |
| Xanthan gum | 2.16 | 4.00 | 0.60 | 2.16 | 2.16 | 2.16 | 2.16 |
| Talc | 0.63 | 0.63 | 0.63 | 0.63 | 0.63 | 0.63 | 0.63 |
| Hydroxypropyl cellulose | 2.51 | 2.51 | 2.51 | 2.51 | 2.51 | 2.51 | 2.51 |
| Concentrated glycerol (Parenthesized value is glycerol concentration relative to the total amount of the gel film) | 0.00 (0%) | 0.00 (0%) | 0.00 (0%) | 0.11 (2.0%) | 0.28 (4.9%) | 0.59 (10.2%) | 0.00 (0%) |
| Titanium oxide | 0.11 | 0.11 | 0.11 | 0.11 | 0.11 | 0.11 | 0.11 |
| Mass ratio of the film relative to the tablet having the film (%) | 3.00 | 4.03 | 2.14 | 3.07 | 3.16 | 3.33 | 3.00 |
| Number of tablet got moldy in stability test among 30 tablets | 0/30 | 0/30 | 0/30 | 0/30 | 6/30 | 11/30 | - |

The amount of each ingredient in the gel film shown in Table 3-1 is the same as the amount of the corresponding ingredient relative to the total amount of solid contents in the coating liquid.

### Stability test

In conditions at 25°C and 75%RH, 30 tablets in each of Examples 3-1 to 3-4 and Comparative Examples 3-1 and 3-2 were allowed to stand for 30 days, and the growth of mold on the surface was visually observed. Of 30 tablets, the number of tablets on which mold was grown is shown together in Table 3-1.

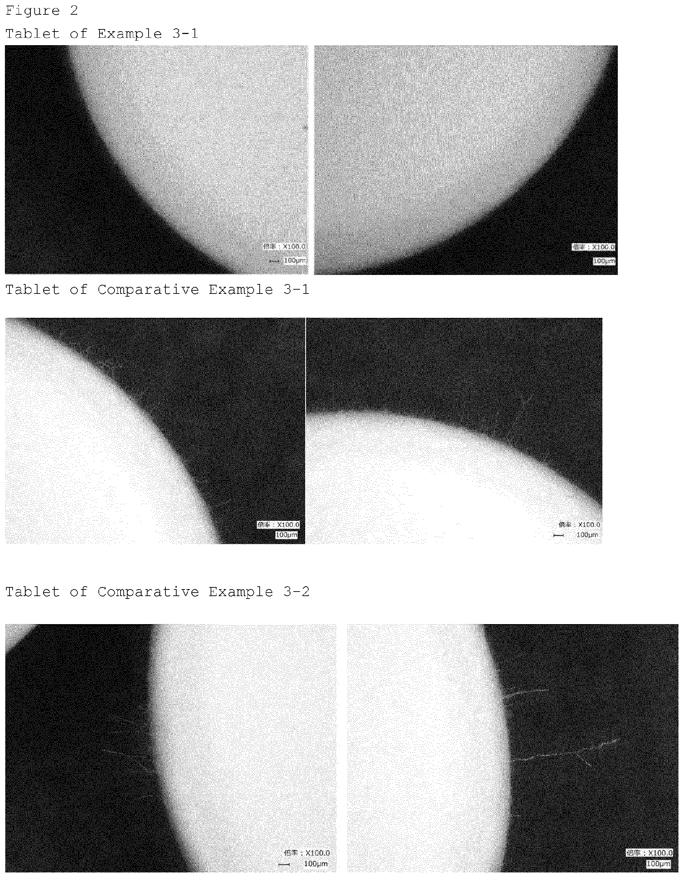

Comparing Examples 3-1 and 3-4 and Comparative Examples 3-1 and 3-2 revealed that the critical point of glycerol concentration in the gel films at which mold was grown was between 2.0% by mass and 4.9% by mass. Photographs of the surfaces of the tablets of Example 3-1, Comparative Example 3-1, and Comparative Example 3-2 are shown in Figure 2. On each surface of the tablets of Comparative Examples 3-1 and 3-2, mold growth was observed, but on the surface of the tablet of Example 3-1, no mold growth was observed.

Example 3-2 contained a larger amount of xanthan gum, which is a polymer gelable on contact with water, than Comparative Example 3-1, but used the gel coating liquid in which xanthan gum was dispersed in a poor solvent, absolute ethanol. Hence, the gel coating liquid did not have an excessively high viscosity and accordingly did not clog nozzles of the coating apparatus. Consequently, the uncoated tablets were able to be coated with the gel film.

### (4) Fourth embodiment

### Production of tablets coated with gel film

In a container-rotating mixer (Tumbler Mixer TM-15S, Showa Kagakukikai), 3.384 kg of D-mannitol (Granutol F, Freund Corporation), 180 g of hydroxypropyl cellulose (HPC-L-FP, Nippon Soda), and 36 g of magnesium stearate (plant-based, Taihei Chemical Industrial) were placed and mixed. The mixture was tableted by using a rotary tableting machine (VIRGO24, Kikusui Seisakusho) to give uncoated tablets having a tablet weight of 180 mg and a tablet thickness of 2.9 mm.

In absolute ethanol, xanthan gum (SAN-ACE S, San-Ei Gen F.F.I), titanium oxide (KRONOS 1171, Kronos Worldwide, Inc.), talc (ML-115, FUJI TALC INDUSTRIAL), and hydroxypropyl cellulose (HPC-SL, Nippon Soda) were dissolved or dispersed to give a gel coating liquid. The uncoated tablets were subjected to film coating by using a coating machine (HICOATER, Freund Corporation), giving tablets coated with a gel film.

The formulae of the gel films of the tablets prepared in Examples 4-1 to 4-6 and Comparative Examples 4-1 to 4-4 are shown in Table 4-1.

**Table 4-1**

| Unit: mg in the gel film of one tablet | | | | | | | | | | |
|---|---|---|---|---|---|---|---|---|---|---|
| Parenthesized value of titanium oxide is % by mass relative to the total amount of the gel film | | | | | | | | | | |
| | Example 4-1 | Example 4-2 | Example 4-3 | Example 4-4 | Example 4-5 | Example 4-6 | Comparative Example 4-1 | Comparative Example 4-2 | Comparative Example 4-3 | Comparative Example 4-4 |
| Xanthan gum | 2.16 | 2.16 | 2.16 | 2.16 | 4.00 | 0.60 | 2.16 | - | 2.16 | 2.16 |
| Titanium oxide | 0.02 (0.4%) | 0.11 (2%) | 0.59 (10%) | 1.33 (20%) | 0.11 (1.5%) | 0.11 (2.9%) | - (0%) | 0.11 | 2.27 (30%) | 5.30 (50%) |
| Talc | 0.63 | 0.63 | 0.63 | 0.63 | 0.63 | 0.63 | 0.63 | 0.63 | 0.63 | 0.63 |
| Hydroxypropyl cellulose | 2.51 | 2.51 | 2.51 | 2.51 | 2.51 | 2.51 | 2.51 | 4.67 | 2.51 | 2.51 |
| Mass ratio of the film relative to the tablet having the film (%) | 2.95 | 3.00 | 3.27 | 3.68 | 4.03 | 2.14 | 2.94 | 3.00 | 4.20 | 5.89 |
| Δ | 46 | 48.4 | 28 | 23.4 | - | - | -1.2 | - | 13.2 | 6.2 |
| Slidability | ○ | ○ | ○ | ○ | ○ | ○ | ○ | × | ○ | ○ |

| | | | | | | | | | | |
|---|---|---|---|---|---|---|---|---|---|---|
| Xanthan gum : SAN-ACE S Titanium oxide : CRONOS 1171 Talc : ML-115 Hydroxypropyl cellulose : HPC-SL | | | | | | | | | | |

### Evaluation of identifiability of printed characters

On each tablet, "pitavastatin (in Japanese)", "2", and "AMEL (in Japanese)" were printed by using a laser irradiation apparatus (company name, model number). The wavelength was set at 355 nm. The expander was set at 6,000 mm, and the pulse energy was set at 40%.

Photographs of the character displaying surfaces on the tablets of Examples 4-1 to 4-6 and Comparative Examples 4-1 to 4-4 were taken under an optical microscope and were converted into 8-bit grayscale images. Gray values (luminance values) at five positions in the character area and the background area on each tablet of Examples 4-1 to 4-4, Comparative Example 4-3, and Comparative Example 4-4 were measured by using ImageJ (ImageJ is a software program which has been developed by the US National Institutes of Health and is generally used), and the average value was calculated. The gray value is a value indicating one of 256 gradations where black is 0 and white is 255. The difference in average gray value between a character area and a background area was used as the readability index. The density scale of a CT device was 8 bits (2⁸ = 256 gradations), and the gray value in terms of 256 gradations is commonly used accuracy. When the difference Δ is 16 or more, characters can be clearly identified (MEDICAL IMAGING TECHNOLOGY Vol.27, No.4, 2009, 258-262).

The difference (Δ) in average gray value between a background area and a character area is show together in Table 4-1.

On the tablet of Comparative Example 4-1, no characters were displayed, and thus gray values (luminance values) were measured at five positions by using ImageJ, and the difference between the maximum value and the minimum value was calculated as Δ.

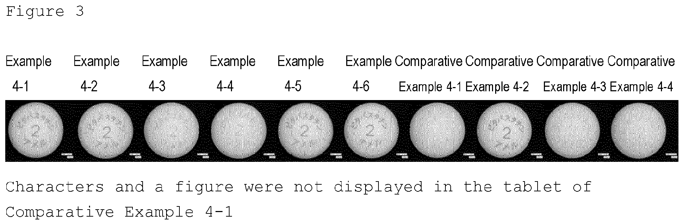

Photographs of the printed face on the tablets of Examples 4-1 to 4-6 and Comparative Examples 4-1 to 4-4 are shown in Figure 3.

When the titanium oxide concentration was 20% by mass or less, the difference in gray value was markedly large as compared to when the concentration was more than 20% by mass. When no titanium oxide was contained, no characters were printed. It is revealed that characters are identifiably displayed when titanium oxide is contained at a concentration of 20% by mass or less. The gel film in Example 4-5 contained xanthan gum in an extremely large amount, whereas the gel film in Example 4-6 contained xanthan gum in an extremely small amount, but characters can be clearly identified.

As compared with the gel film in Example 4-1, the gel film in Comparative Example 4-2 contained no xanthan gum but contained hydroxypropyl cellulose in an amount to compensate the xanthan gum. On the tablet of Comparative Example 4-2, characters were clearly printed.

### Evaluation of gel film slidability

The slidability of each tablet of Examples 4-1 to 4-6 and Comparative Examples 4-1 to 4-4 was evaluated on an acrylic plate by the following method.

An acrylic plate was placed at an angle of 20° to the horizontal plane. The bottom face of a tablet was slightly wetted with a damp sponge, and the tablet was placed on the upper part of the acrylic plate. From a position higher than the placed tablet (from the upstream side), about 50 mL of water was allowed to slowly flow, and whether the tablet flowed was evaluated. A tablet flowing with running water was evaluated as ∘, and a tablet not flowing was evaluated as ×.

The results are shown together in Table 4-1. The tablet of Comparative Example 4-2 containing no xanthan gum had poor slidability, but the tablets of Examples 4-1 to 4-6 and Comparative Examples 4-1, 4-3, and 4-4 containing xanthan gum had good slidability.

### Industrial Applicability

The oral solid composition in the first embodiment in which an oral solid preparation such as tablets is coated with a rapidly disintegrating gel film has a surface that becomes smooth with a small amount of saliva in the oral cavity and is easily swallowed even by a patient with difficulty in swallowing. Hence, the oral solid composition has a high utility value in terms of improvement in medication adherence of patients with difficulty in swallowing. Moreover, the gel film rapidly disintegrates and disappears in the gastrointestinal tract and thus does not interfere with the pharmaceutical effect of an active ingredient.

A common oral solid composition coated with a gel film is likely to discolor when stored, but the oral solid composition in the second embodiment is unlikely to discolor even when stored. The oral solid composition is therefore highly practical.

A common oral solid composition coated with a gel film is likely to get moldy, but on the oral solid composition in the third embodiment, mold growth is suppressed. Even when a polymer gelable on contact with water is contained at a larger content to improve the ease of swallowing, a coating apparatus is not clogged, and coating can be completed within an appropriate time period.

The oral solid composition comprising a gel film in the fourth embodiment is coated with the gel film, but characters, numeric characters, symbols, and the like can be clearly printed thereon by ultraviolet laser irradiation.

## Claims

1. A rapidly disintegrating gel film comprising a polymer gelable on contact with water, a carbonate, and an acidic substance.

2. The rapidly disintegrating gel film according to claim 1, in which the carbonate is contained at 0.5 to 60% by mass relative to the total amount of the rapidly disintegrating gel film.

3. The rapidly disintegrating gel film according to claim 1 or 2, in which the acidic substance is contained at 0.5 to 40% by mass relative to the total amount of the rapidly disintegrating gel film.

4. The rapidly disintegrating gel film according to any one of claims 1 to 3, in which the acidic substance is contained at 0.1 to 1 part by mass relative to 1 part by mass of the carbonate.

5. An oral solid composition comprising the rapidly disintegrating gel film according to any one of claims 1 to 4 and an oral solid preparation.

6. The oral solid composition according to claim 5, in which the oral solid preparation is a tablet.

7. A coating solid composition comprising a polymer gelable on contact with water, a carbonate, and an acidic substance.

8. The coating solid composition according to claim 7, in which the polymer gelable on contact with water is a polysaccharide.

9. The coating solid composition according to claim 7 or 8, in which the concentration of the polymer gelable on contact with water is 16 to 56% by mass relative to the total amount of the coating solid composition.

10. The coating solid composition according to any one of claims 7 to 9, in which the concentration of the carbonate is 0.5 to 60% by mass relative to the total amount of the coating solid composition.

11. The coating solid composition according to any one of claims 7 to 10, in which the concentration of the acidic substance is 0.5 to 40% by mass relative to the total amount of the coating solid composition.

12. The coating solid composition according to any one of claims 7 to 11, in which the content of the acidic substance is 0.1 to 1 part by mass relative to 1 part by mass of the carbonate.

13. An oral solid composition comprising a gel film containing a polymer gelable on contact with water and comprising an oral solid preparation, the oral solid composition being hermetically packed or being tightly packed together with a desiccant.

14. The oral solid composition according to claim 13, in which the oral solid composition is hermetically packed in a plastic bottle, a bag molded from a plastic sheet, or a bag molded from a sheet comprising an aluminum layer or is tightly packed in a plastic bottle.

15. The oral solid composition according to claim 13 or 14, in which the oral solid composition packaged in a PTP package or an SP package is hermetically packed or is tightly packed together with a desiccant.

16. The oral solid composition according to claim 14 or 15, in which the aluminum layer of the bag molded from a sheet comprising an aluminum layer has a thickness of 5 to 40 µm.

17. The oral solid composition according to any one of claims 13 to 16, in which the hermetically packed oral solid composition is hermetically packed together with a desiccant.

18. The oral solid composition according to any one of claims 13 to 17, in which the polymer gelable on contact with water is a polysaccharide.

19. The oral solid composition according to any one of claims 13 to 18, in which the oral solid preparation is a tablet.

20. A gel film formed by using a coating liquid containing a polymer gelable on contact with water, in which the concentration of glycerol is 2% by mass or less relative to the total amount of the gel film, and the polymer gelable on contact with water is partially or practically completely dispersed in the coating liquid.

21. The gel film according to claim 20, in which the polymer gelable on contact with water is a polysaccharide.

22. The gel film according to claim 20 or 21, in which the coating liquid contains a polar solvent other than water.

23. The gel film according to any one of claims 20 to 22, further comprising a carbonate and/or an acidic substance.

24. An oral solid composition comprising the gel film according to any one of claims 20 to 23 and an oral solid preparation.

25. The oral solid composition according to claim 24, in which the oral solid preparation is a tablet.

26. A coating liquid comprising a polymer gelable on contact with water, in which the concentration of glycerol is 2% by mass or less relative to the total amount of the solid content in the coating liquid, and the polymer gelable on contact with water is partially or practically completely dispersed in the coating liquid.

27. The coating liquid according to claim 26, in which the polymer gelable on contact with water is a polysaccharide.

28. The coating liquid according to claim 26 or 27, in which the coating liquid contains a polar solvent other than water.

29. The coating liquid according to any one of claims 26 to 28, further comprising a carbonate and/or an acidic substance.

30. A gel film comprising a polymer gelable on contact with water and comprising titanium oxide (TiO₂) at 20% by mass or less relative to the total amount of the gel film, in which marking is provided on the surface of the gel film without engraving or any coloring agent.

31. The gel film according to claim 30, in which the concentration of titanium oxide (TiO₂) in the gel film is 0.1% by mass or more relative to the total amount of the gel film.

32. The gel film according to claim 30 or 31, in which the polymer gelable on contact with water is a polysaccharide.

33. The gel film according to any one of claims 30 to 32, further comprising a carbonate and/or an acidic substance.

34. The gel film according to any one of claims 30 to 33, in which the marking is provided on the surface of the gel film by ultraviolet laser irradiation.

35. The gel film according to any one of claims 30 to 34, formed by using a coating liquid in which the polymer gelable on contact with water is partially or practically completely dispersed.

36. An oral solid composition comprising the gel film according to any one of 30 to 35 and an oral solid preparation.

37. The oral solid composition according to claim 36, in which the oral solid preparation is a tablet.

38. The oral solid composition according to claim 36 or 37, in which marking is provided on the surface of the oral solid composition by ultraviolet laser irradiation.

39. A coating composition for forming a gel film having a surface with marking, the coating composition comprising a polymer gelable on contact with water and comprising titanium oxide at 20% by mass or less relative to the total amount of the coating composition.

40. The coating composition according to claim 39, in which the polymer gelable on contact with water is a polysaccharide.

41. The coating composition according to claim 39 or 40, further comprising a carbonate and/or an acidic substance.
